# FASCICULE DE BREVET EUROPEEN

(11) **EP 0 293 357 B1**
(45) Date de publication et mention de la délivrance du brevet: **05.05.1993**
(21) Numéro de dépôt: 88870095.2
(22) Date de dépôt: 24.05.1988
(51) Int. Cl.: C07K 15/00

(54) **Expression de la proapolipoprotéine A-I humaine**
Expression von menschlichem Proapolipoprotein A-1
Expression of human proapolipoprotein A-1

(30) Priorité: 28.05.1987 GB 8712540
(43) Date de publication de la demande: 30.11.1988
(73) Titulaire: U C B, S.A., 1050 Bruxelles (BE)
(72) Inventeur: Bollen, Alex, B-1711 Itterbeek (BE); Gobert, Jean, B-1040 Bruxelles (BE); Wülfert, Ernst, B-1180 Bruxelles (BE)
(74) Mandataire: Dusseldorp, Raymond

(56) Documents cités:
- EP-A- 0 267 703
- WO-A-86/05810
- FEBS LETTERS, vol. 194, no. 2, janvier 1986, pages 343-346, Federation of European Biochemical Societies; R. LORENZETTI et al.: "Expression of the human apolipoprotein AI gene fused to the E. coli gene for beta-galactosidase"
- NUCLEIC ACIDS RESEARCH, vol. 13, no. 6, mars 1985, pages 1923-1938, IRL Press Ltd, GB; G. BUELL et al.: "Optimizing the expression in E. coli of a synthetic gene encoding somatomedin-C (IGF-I)"

## Description

La présente invention se rapporte à de nouvelles séquences d'ADN recombinantes comprenant une séquence codant pour la proapolipoprotéine A-I humaine, à des vecteurs de clonage et d'expression contenant ces séquences d'ADN, aux cellules hôtes transformées avec ces vecteurs d'expression, ainsi qu'aux procédés de production de la proapolipoprotéine A-I humaine en utilisant les nouvelles séquences d'ADN recombinantes, les vecteurs et transformants.

L'apolipoprotéine A-1 (apo A-1) humaine est le constituant protéinique principal des lipoprotéines de haute densité (LHD) et des chylomicrons de la lymphe. Le foie et l'intestin grêle sont les centres primaires de la synthèse de l'apo A-I. L'apo A-I est synthétisée dans ces organes sous la forme d'un précurseur protéinique (preproapo A-I). Un clivage cotraductionnel du prépeptide intervient à l'intérieur de la cellule et le proapo A-I est sécrétée dans le plasma et dans la lymphe.

Le proapo A-I contient six acides aminés supplémentaires (Arg - His - Phe - Trp - Gln - Gln) qui sont fixés au groupe amino terminal de l'apo A-I. En atteignant la région vasculaire, la proapo A-I est clivée in vivo par une enzyme protéolytique spécifique (apo A-I propeptidase) pour donner l'apo A-I mature.

L'apo A-1 mature est un polypeptide unique non glycosylé de séquence connue, composé de 243 acides aminés (H.B. BREWER et coll., Biochem.Biophys.Res.Commun.80,(1978),623-630); il sert de cofacteur pour l'enzyme plasmatique de la lécithine: cholestérol acyltransférase, qui est responsable de la formation dans le plasma de la plupart des esters du cholestérol. Des défauts dans les structures ou dans la biosynthèse de l'apolipoprotéine peuvent conduire à des désordres dans le système de transport des lipides plasmatiques et au développement d'une maladie des artères coronaires. On a montré que de faibles taux plasmatiques en apo A-I et en LHD constituent un facteur de risque important pour des crises cardiaques (infarctus du myocarde) et pour d'autres maladies athérosclérotiques vasculaires. Des mutations dans le gène codant pour l'apo A-I en particulier, ont été associées à des réductions du taux de LHD ainsi qu'à des maladies prématurées des artères coronaires.

L'apo A-1 et les LHD sont les constituants majeurs du plasma qui participent au transfert du cholestérol des tissus périphériques (artères) vers le foie (appelé aussi transport inverse du cholestérol) pour être excrété par l'organisme. Etant donné que l'accumulation du cholestérol dans les artères est la caractéristique et le mécanisme le plus important de l'athérosclérose, la stimulation du transport inverse du cholestérol au moyen de l'apo A-1 pourrait retarder et inverser le processus athérosclérotique et par là diminuer l'incidence des crises cardiaques.

La maturation de la proapo A-I en apo A-I peut se produire quantitativement à l'extérieur de la cellule avec un temps de séjour dans le sang inférieur à 12 heures. Etant donné que la proapo A-I est le précurseur majeur, si pas le seul, de l'apo A-I mature, il pourrait être utilisé dans une thérapeutique de substitution chaque fois que les taux de LHD diminuent, par exemple dans des déficiences héréditaires ou acquises. A cause de l'utilité thérapeutique de l'apo A-I en général, les chercheurs ont recherché des techniques qui permettent de produire l'apo A-I en grandes quantités. Traditionnellement, de telles techniques comportent la purification de l'apo A-I à partir du plasma sanguin.

Plusieurs publications (P. CHEUNG et L. CHAN, Nucleic Acids Res.11,(1983),3703-3715; J.J. SEILHAMER et coll., DNA,3,(1984),309-317 et la demande de brevet japonaise n 96998/86) ont montré que l'ADN complémentaire codant pour la préproapo A-I peut être préparé par des techniques de génie génétique bien connues.

R. LORENZETTI et coll., (FEBS Lett.194,(1986),343-346) ont montré que l'apo A-I peut être exprimée dans E. coli sous la forme d'une protéine fusionnée à la beta-galactosidase, non clivable. Cependant, des tentatives pour exprimer l'apo A-I mature sous la forme d'une protéine non fusionnée sont restées sans succès.

Dans la demande de brevet japonaise n° 96998/86 citée ci-dessus, on décrit l'expression d'une protéine semblable à l'apolipoprotéine A-I humaine, dans E. coli qui a été transformé par un plasmide pHAIE-I contenant le gène de structure de l'apo A-I sous le contrôle du promoteur tac. Cependant, le gène de structure est incomplet et se compose des codons pour les acides aminés + 4 à +243, précédés par un codon ATG d'initiation de la traduction. Il en résulte que le produit d'expression obtenu contient la méthionine N-terminale (correspondant au codon ATG) qui peut provoquer des effets secondaires lors de son emploi en thérapeutique.

J.B. MALLORY et coll. (J.BioLChem.262,(1987),4241 -4247; demandes de brevet internationales PCT WO 86/04920 et WO 87/02062) décrivent l'expression de l'apolipoprotéine A-I humaine dans des cellules ovariennes d'hamsters chinois (culture de cellules animales). La construction utilisée contient le gène complet de la préproapolipoprotéine A-I humaine. Les cellules ovariennes d'hamsters chinois semlent transformer la forme proapo en la forme apo mature, puisque seulement 5 à 10% de la protéine apo A-I sécrétée est de la proapo A-I, le restant étant la protéine apo A-I mature. La productivité du système est relativement faible, puisque 0,5x10⁶ cellules sécrètent seulement 25-30 µg/ml d'apo A-I en 24 heures. Dans la demande de brevet internationale PCT WO 87/02062, quelques exemples de réalisation se rapportent à l'expression de l'apolipoprotéine A-I humaine dans d'autres hôtes, tels que E. coli (hôte bactérien) et S. cerevisiae (levure). Cependant, aucune des constructions employées ne contient l'informa - tion génétique pour la forme proapo et la protéine exprimée n'est pas la proapolipoprotéine A-I humaine.

La présente invention concerne les moyens et les méthodes pour préparer la proapolipoprotéine A-I humaine par la technologie de l'ADN recombinant, c'est-à-dire la protéine proapo A-I mature qui est susceptible d'être clivée par l'enzyme protéolytique spécifique apo A-1 propeptidase. Par l'expression "mature" telle qu'elle est utilisée ici, on entend non seulement la proapo A-I humaine telle quelle, mais aussi la protéine correspondante dont le premier acide aminé est la méthionine et dont la présence est due au codon ATG d'initiation de la traduction dans la construction du vecteur d'expression.

La présente invention se rapporte à la construction de vecteurs de clonage et d'expression contenant une séquence d'ADN codant pour la proapo A-I humaine, de manière à permettre l'amplification et par conséquent l'expression de la protéine proapo A-I humaine mature aussi bien que la met-proapo A-I, ses conjugués de fusion ou ses conjugués avec le signal N-terminal. De même, la présente invention concerne des cultures de cellules viables ou des microorganismes génétiquement modifiés parce qu'ils hébergent de tels vecteurs, et capables de produire le polypeptide proapo A-I humaine. En outre, la présente invention procure la proapo A-I humaine dans un état physique distinct de celui que l'on trouve ou que l'on isole à partir d'une source ou d'un environnement naturels; en raison de sa méthode de préparation, la proapo A-I humaine est essentiellement débarrassée des protéines endogènes habituelles et d'autres matériaux ou substances d'origine.

La présente invention concerne la production de la proapo A-I humaine au moyen de la technique de l'ADN recombinant dans tous ses aspects et ne doit pas être interprêtée comme étant limitée à des détails spécifiques décrits et englobées dans le cadre de l'invention.

Un aspect fondamental de la présente invention concerne la production d'une protéine qui consiste en ou contient la proapo A-I humaine mature et qui, dès lors, peut être convertie, in vitro et in vivo, en apo A-I humaine mature par l'action protéolytique de l'apo A-I propeptidase. Le produit proapo A-I humain peut être utilisé tel quel pour des usages thérapeutiques; on peut utiliser également la met-proapo A-I, si la présence du radical méthionyle est pharmaceutiquement acceptable, parce que ce produit peut être converti naturellement et efficacement dans la circulation sanguine par la propetidase naturelle pour fournir de l'apo A-I humaine mature authentique. Si on le désire, la proapo A-I humaine peut être produite dans des microorganismes ou dans des cultures de cellules sélectionnées qui sont capables de traiter la méthionine N-terminale et qui, par conséquent, sont capables de produire la proapo A-I humaine sous une forme qui ne contient pas la méthionine N-terminale. Alternativement, qu'elle contienne ou non un radical méthionyl N-terminal, la proapo A-I humaine peut être clivée in vitro pour obtenir l'apo A-I humaine mature, qui peut être utilisée pour des applications thérapeutiques. Il en est de même pour les conjugués de fusion et les conjugués incluant le signal N-terminal de la proapo A-I; le clivage produit l'apo A-I humaine authentique dépourvue de la méthionine N-terminale.

Un second aspect important de la présente invention réside dans l'emploi d'une séquence modifiée codant pour au moins une partie de la molécule de la proapo A-I humaine; cette séquence codante modifiée améliore l'efficacité de la traduction en raison de la réduction ou même de la prévention de la formation de structures en épingle. Il est possible que R.LORENZETTI et coll. (FBS Lett.194,(1986),343-346) n'aient pas pu observer l'expression d'une protéine apo A-I mature non fusionnée parce que leurs constructions d'ADN étaient susceptibles de former de manière appréciable des structures en épingle, conduisant à une expression très inefficace. On a constaté avec surprise qu'une expression efficace peut être assurée par une modification appropriée de quelques codons dans la séquence codant pour les acides aminés -6 à +14 de la proapo A-1 humaine. L'expression "modification appropriée" telle qu'elle est utilisée ici implique que quelques - uns des codons naturels sont remplacés par d'autres codons qui, selon le code génétique, représentent les mêmes acides aminés, et elle implique, en outre, que toutes les modifications prises ensemble conduisent à la réduction ou même à la prévention de la formation de structures en épingle. Il est important de signaler que les modifications de la séquence d'ADN n'ont aucun effet sur la nature du site de clivage de la propeptidase, vu que la séquence d'acides aminés reconnue par la propeptidase est préservée dans la construction.

Au niveau des protéines, la présente invention se rapporte à la proapo A-I humaine en tant que produit d'une culture de cellules ou d'un microorganisme génétiquement modifié. Cette proapo A-I humaine peut se présenter sous la forme d'une proapo A-I mature, sous la forme d'une proapo A-I mature précédée par un résidu de méthionine, sous la forme d'une protéine fusionnée telle que par exemple beta-galactosidase-proapo A-I et sous la forme du composé préproapo A-I. Le produit obtenu est essentiellement débarrassé des protéines endogènes habituelles et d'autres matériaux ou substances d'origine commune à la source naturelle (plasma de sang) de la protéine concernée. La culture de cellules ou le microorganisme utilisé pour la production n'est pas limité à des lignées cellulaires et à des organismes spécifiques: tant les cellules procaryotes que les cellules eucaryotes peuvet être employées, y compris les lignées cellulaires animales et humaines. Seulement à titre illustratif, nous donnons ici l'exemple de l'expression dans la bactérie E. coli (comme représentant des cellules procaryotes) et dans la levure Saccharomyces cerevisiae ainsi que dans des cellules d'insectes infectées par baculovirus (comme représentants des cellules eucaryotes).

Au niveau des protéines, la présente invention se rapporte également à l'apo A-I humaine obtenue par le traitement par l'apo A-I propeptidase de la proapo A-I humaine préparée ci-dessus. Cette apo A-I humaine sera identique à la forme authentique de l'apo A-I mature humaine et sera dépourvue de tout résidu de méthionine N-terminal.

Au niveau de l'application, la présente invention se rapporte aux compositions pharmaceutiques renfermant la proapo A-I humaine et/ou l'apo A-I humaine ci-dessus, et contenant en outre, au moins un véhicule pharmaceutiquement acceptable, un solvant, un diluant ou un excipient convenablement choisi selon la voie d'administration et les exigences habituelles dans la formulation des compositions pharmaceutiques.

Au niveau de l'ADN, la présente invention se rapporte à une séquence d'ADN recombinante compre - nant une séquence qui code pour la proapolipoprotéine A-I humaine dans laquelle une partie de la séquence codante naturelle a été remplacée par un fragment d'ADN codant pour les mêmes acides aminés mais consistant en une séquence de nucléotides différente, de manière à réduire ou à prévenir la formation de structures en épingle. Selon une forme de réalisation spécifique préférée, la séquence naturelle codant pour les acides aminés -6 à +14 de la proapo A-I a a été remplacée par la séquence (un seul brin indiqué): 5' - ATGAGACATTTCTGGCAGCAGGACGAACCTCCACAATCTCCTTGGGATAGAGTTAAGGACTTG - 3' ladite séquence comprenant un codon supplémentaire ATG d'initiation de la traduction et des codons modifiés pour les résidus des acides aminés - 6, - -1, + 1, + 3, + 4, + 5, + 6, + 7, + 10, + 11 et + 14.

Sur le plan de l'ADN, la présente invention se rapporte également à des vecteurs de clonage réplicables et à des vecteurs d'expression comprenant la séquence d'ADN recombinante précitée qui code pour la proapo A-I humaine. La présente invention se rapporte plus particulièrement aux plasmides recombinants pULB9291, pULB9292, pULB9296, pULB9299, pNIV1612 et pNIV1613 dont la construction est décrite ci-après. Les premiers plasmides cités, pULB9291 et pULB9292 contiennent le gène de structure modifié de la proapo A-I, précédé par un codon ATG et sous le controle du promoteur P_{L} du phage lambda. Ces plasmides sont des vecteurs d'expression qui conviennent pour E. coli et commandent la synthèse de la proapo A-I humaine, qui peut être clivée par la propeptidase pour engendrer une apo A-I humaine mature authentique. Lorsqu'il est introduit dans E. coli, le plasmide pULB9296 commande l'expression d'une protéine fusionnée contenant la betagalactosidase et le proapo A-I humaine sous le contrôle de la région du promoteur lac de E. coli. Etant donné que le produit de fusion contient encore la séquence de clivage de la propeptidase, il peut être clivé pour fournir de l'apo A-I mature humaine authentique.

Le plasmide pULB9299 est un vecteur d'expression qui convient pour des espèces de levures et contient les régions du promoteur et du terminateur de la transcription de l'ARG3 pour assurer une expression efficace dans une levure. A nouveau, la proapo A-I humaine obtenue peut être scindée par la propeptidase pour fournir de l'apo A-I humaine mature authentique.

Le plasmide pNIV1612 contient le gène de structure de la proapo A-I fusionné à la séquence d'ADN du peptide signal de la protéine OmpA de E. coli et sous le contrôle du promoteur Ipp (lipoprotéine) et du promoteur-opérateur lac. Dans la construction, la séquence codante pour le peptide signal de ompA précède la séquence de la proapo A-I sans le codon ATG d'initiation de la traduction. Le plasmide est un vecteur de sécrétion approprié pour E. coli et commande la synthèse de la proapo A-I humaine qui peut être sécrétée dans le périplasme sans la méthionine N-terminale. Le plasmide pNIV1613 est un vecteur de transfert pour l'introduction de la séquence de l'ADNc de la proapo A-I humaine dans le baculovirus. Il comprend le promoteur polyhédrine du baculovirus et le gène de structure de la proapo A-I, y compris le codon ATG d'initiation de la traduction. Conjointement avec la souche sauvage de baculovirus (Autographa californica nuclear polyhedrosis virus, AcNPV), le plasmide pNIV1613 commande la synthèse de la proapo A-I dans des cellules d'insectes et peut se retrouver débarrassée de la méthionine après des modifications post-traductionnelles dans les cellules d'insectes.

Finalement, la présente invention se rapporte également à des cultures de cellules et à des microorganismes qui ont été transformés par un vecteur de clonage ou par un vecteur d'expression tel que décrit ci-dessus, et en particulier, à des cultures de cellules et à des microorganismes transformés qui sont capables de produire de la proapo A-I humaine. L'expression "transformés" telle qu'elle est utilisée ici, prend le sens large de "génétiquement modifiés" et n'est certainement pas limitée au concept étroit de "transformation bactérienne". Dépendant de la nature du vecteur employé et de l'hôte sélectionné, toute technique de génie génétique compatible pour obtenir la modification génétique désirée, peut être utilisée, y compris la transfection, la traduction, etc.

Les cultures de cellules et les microorganismes préparés selon l'invention peuvent être utilisés pour une production par fermentation à l'échelle industrielle de la proapolipoprotéine A-I humaine.

L'invention décrite ici a été réalisée en employant entre autres la souche MM 294 du microorganisme E. coli K12 (endoA thi -, hsdR, supE); cette souche a été déposée à l'Americain Type Culture Collection (ATCC No. 33625) sans restriction en ce qui concerne son accessibilité. Toutefois, différentes autres souches microbiennes sont utilisables, comprenant des souches connues de E. coli telles que E. coli B, E. coli B, E. coli x 1776 (ATCC No. 31537, déposée le 3 juillet 1979, sans restriction), E. coli AR58, dérivée de N99 (ATCC No. 33956) avec clll-, cl 857, bio-, fonctions delta H dépourvues de lambda, vendue par PL-PHARMACIA (J.E. MOTT et coll., Proc.Natl.Acad.Sci.USA,82,(1985),88-92; G. DEVARE et coll., Cell,36,-(1984),43-49), E. coli JM101 (ATCC No 33876) (J. MESSING et coll., Nucleic Acids Res.9,(1981),309-321), ou E. coli JA221 (Ipp-, hdsM⁺, trpE5, leuB6, lacY, recAl/F', lacl^{q}, lac+, pro+) (J. GHRAYEB et coll., EMBO J.3,(1984),2437-2442), ou d'autres souches microbiennes dont beaucoup sont déposées et accessibles auprès d'institutions de dépôts de microorganismes reconnues, telles que l'American Type Culture Collection (ATCC) - - cf. les listes du catalogue ATCC. Ces autres microorganismes comprennent par exemple, Bacilli tels que Bacillus subtilis et d'autres Enterobactériacées parmi lesquelles on peut citer, par exemple, Salmonella Typhimurium et Serratia marcesans, utilisant des plasmides qui peuvent se répliquer et exprimer des séquences de gènes hétérologues.

Les plasmides d'expression pour usage chez les bactéries, par exemple E. coli, sont habituellement obtenus à partir du plasmide pBR322 utilisé comme vecteur (déposé à l'ATCC sous le numéro d'accès 37017) et en insérant de manière appropriée, la séquence du gène hétérologue en même temps que les signaux d'initiation et de terminaison de la traduction, dans la phase de lecture correcte avec un promoteur fonctionnel, en tirant avantage de sites de restriction naturels ou créés synthétiquement. Le vecteur portera un ou plusieurs gènes caractéristiques de sélection phénotypique et une origine de réplication pour assurer l'amplification dans l'hôte. A nouveau, l'insert hétérologue peut être aligné de manière à être exprimé en même temps qu'une pré-séquence fusionnée, provenant par exemple des gènes du système lac.

Les vecteurs d'expression pour baculovirus, par exemple pAcRP6 et pAcYM1 (Y. MATSUURA et coll., J.Gen.Virol.68,(1987),1233 1250) et le baculovirus type sauvage (Autographa californica nuclear polyhe - drosis virus, AcNPV) sont largement utilisés à l'heure actuelle. Ils sont décrits en détail dans la littérature et peuvent être obtenus principalement auprès de TEXAS AGRICULTURAL EXPERIMENT STATION.

Selon la présente invention, on peut aussi utiliser différentes cellules d'insectes comme hôte pour des vecteurs d'expression compatibles, telles que par exemple des cellules de Spodoptera frugiperda Sf9 (M.D. SUMMER et G.E. SMITH. A Manual of Methods for Baculovirus Vectors and Insect cell culture Procédures, Texas University, College Station, (1987); ATCC CRL 1711).

Selon la présente invention, on peut aussi utiliser différentes souches de levures hébergeant des vecteurs d'expression compatibles tels que le plasmide YRp7 (D.T. STINCHCOMB et coll., Nature,282, - (1979),39-43) qui est capable de sélection et de réplication à la fois dans E. coli et dans la levure, en particulier Saccharomyces cerevisiae.

Des souches de levures qui peuvent être utilisées sont la souche RH218 (G. MIOZZARI et coll., J.Bacteriol.134,(1978),48-59) déposée à l'American Type Culture Collection sans restriction (ATCC No. 44076), la souche 10S44c (T. CABEZON et coll., Proc.Natl.Acad.Sci.USA,81,(1984),6594-6598) qui a le génotype leu2 - 3, leu2 -112, pep 4-3 (M. HOYLAERTS et coll., FEBS Lett.204,(1986),83-87) et la souche Ic 1697d (argJ, leu2 - 1) bradytrophe pour l'arginine (ATCC No. 20631).

Pour exprimer un gène hétérologue tel que l'ADNc pour la proapo A-I humaine dans une levure, il est nécessaire de construire un vecteur plasmidique contenant quatre composants.

Le premier composant est le fragment qui permet la transformation à la fois de E. coli et de la levure et qui doit par conséquent contenir un gène de sélection provenant de chaque organisme. Ce peut être le gène responsable de la résistance à l'ampicilline provenant de E. coli (cf. "AmpA") et le gène leu2 provenant de la levure. Ce composant requiert également une origine de réplication provenant de chaque organisme pour être retenu comme ADN plasmidique dans les deux organismes. Ce peut être l'origine de E. coli provenant de pBR322 et l'origine arsl du chromosome III de la levure ou l'origine de réplication de l'ADN circulaire 2µ.

Le second composant du plasmide est une séquence située en 5' d'un gène de levure fortement exprimé pour promouvoir la transcription d'un gène de structure placé en aval. La séquence située en 5' peut être celle provenant des gènes TDH3 ou ARG3 de la levure. Le fragment est construit de manière à éliminer les séquences de structure de TDH3 ou ARG3 qui sont remplacées par une séquence contenant des sites de restriction alternatifs tels que les sites de restriction Ncol ou BamHl, pour le rattachement commode de cette séquence située en 5' au gène de structure.

Le troisième composant du système est un gène de structure construit de telle manière qu'il contient à la fois un signal ATG d'initiation de la traduction et un signal de terminaison de la traduction.

Le quatrième composant est une séquence d'ADN de levure contenant la séquence située en 3' d'un gène de levure qui renferme les signaux appropriés de terminaison de la transcription et de polyadényla- tion. Par exemple, des plasmides commandant la production de la proapo A-I humaine dans la levure peuvent être construits en insérant des fragments de gène pour le polypeptide proapo A-I humain dans le site BamHl du plasmide d'expression pRIT 10774 décrit dans la demande de brevet européen No. 151102.

Des caractéristiques supplémentaires de l'invention apparaîtront encore au cours de la description qui suit de constructions préférées de vecteurs contenant une séquence d'ADN recombinante selon l'invention et des conditions dans lesquelles ces vecteurs sont utilisables. Il sera fait référence à cette occasion aux dessins dans lesquels:

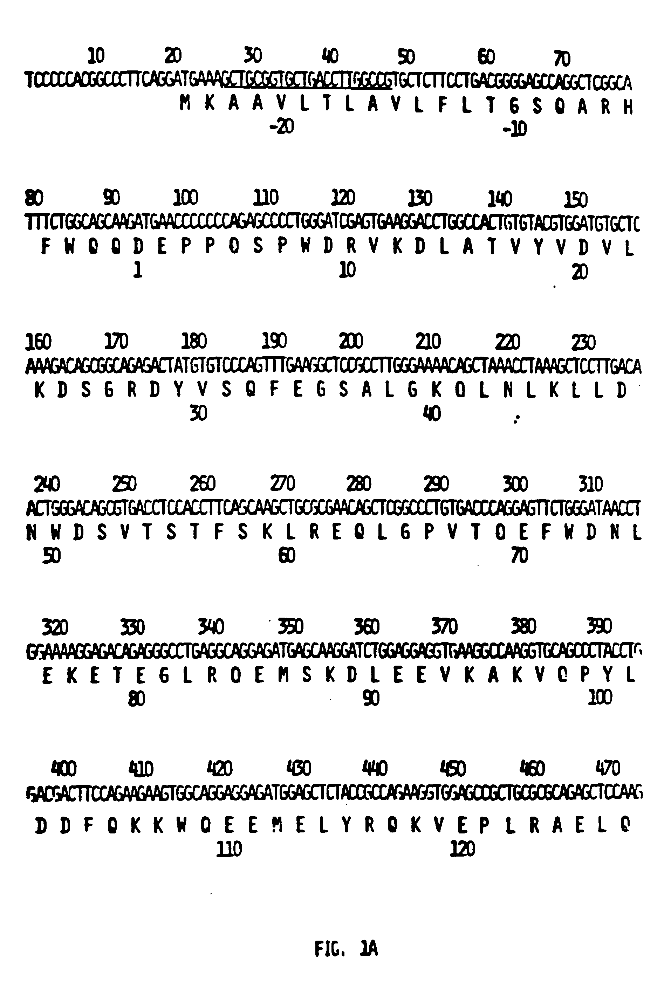

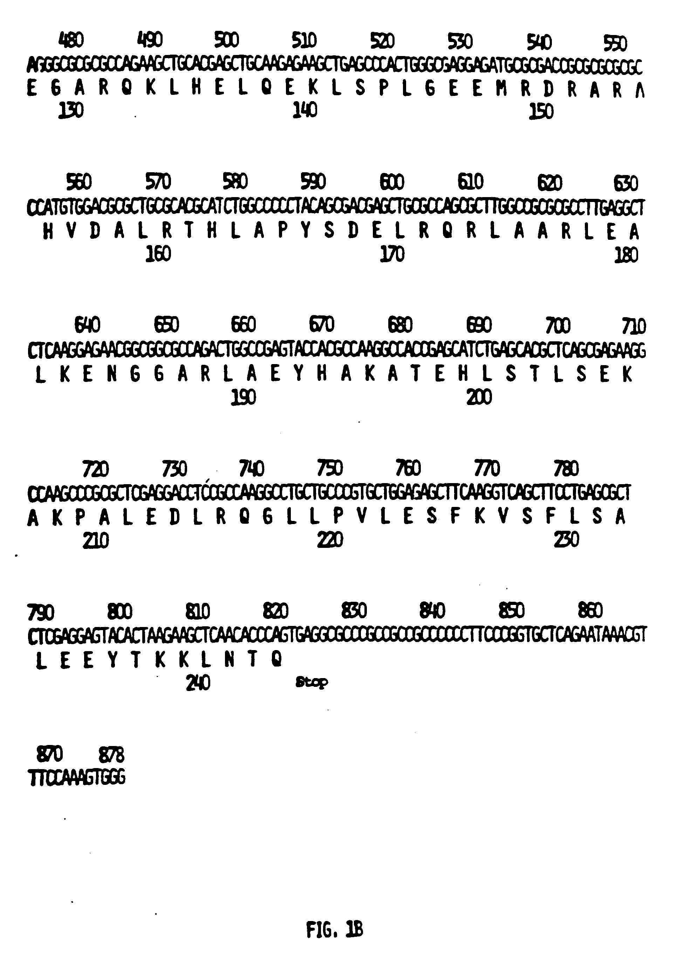

- les figures 1A et B montrent la séquence des nucléotides et des acides aminés de la préproapo A-I humaine. La séquence des nucléotides de l'ARN messager de la préproapo A-I humaine a été déterminée à partir de l'analyse de la séquence d'ADN de l'ADNc du clone pULB1609. Les acides aminés prévus dans le peptide signal, dans le propeptide et dans le polypeptide apo A-I mature sont indiqués et sont numérotés à partir du premier résidu d'acide aminé de la protéine apo A-I. On a souligné la région correspondant à la sonde d'ADN synthétique utilisée pour isoler le clone. Les abréviations par une lettre unique utilisées pour désigner les acides aminés sont les suivantes: A, alanine; C, cystéine; D, acide aspartique; E, acide glutamique; F, phénylalanine; G, glycine; H, histidine; I, isoleucine; K, lysine; L, leucine; M, méthionine; N, asparagine; P, proline Q, glutamine; R, arginine; S, sérine; T, thréonine; V, valine; W, tryptophane et Y, tyrosine.
- la figure 2 représente le schéma de synthèse d'un adapteur oligonucléotidique pour la construction d'un fragment d'ADN codant pour les 6 acides aminés du propeptide et pour les 14 premiers acides aminés du polypeptide apo A-I mature, avec le codon ATG d'initiation. Les flèches indiquent les oligonucléotides utilisés pour synthétiser le fragment Ncol - Ball de 65/61 paires de bases (pb).
- la figure 3 montre la construction du plasmide pULB9291 qui porte les régions régulatrices de P_{L} lambda et la séquence des nucléotides de la proapo A-I. 1.
- la figure 4 montre la construciton du plasmide pULB9296 qui porte le promoteur lac de E. coli et une séquence de nucléotides de betagalactosidase fusionnée avec celle de la proapo A-I.
- la figure 5 montre la construction du plasmide pULB9299 qui porte les régions régulatrices de l'ARG3 de la levure et la séquence des nucléotides de la proapo A-I. 1.
- les figures 6A, B et C montrent la construction du plasmide pNIV1612 qui porte les régions régulatrices lac et Ipp, la séquence codante pour le peptide signal de ompA et la séquence des nucléotides de la proapo A-I. 1.
- la figure 7 montre la construction du plasmide pNIV1613 qui renferme la région régulatrice du gène de la polyhédrine et la séquence des nucléotides de la proapo A-I. 1.

### Description détaillée de l'invention.

Préparation de l'ARN: l'ARN total de foie humain est préparé par la méthode au chlorure de guanidinium (R.A. COX, Methods Enzymol.Xll, part B,(1968),120-129). Cette préparation d'ARN total est ensuite passée sur une colonne d'oligo(dT)-cellulose pour obtenir les polyA⁺ARNs totaux (T. MANIATIS et coll., dans Molecular Cloning (Cold Spring Harbor Laboratory, Cold Spring Harbor, New York, 1982). A partir de 10 g de foie humain on obtient 200 µg de polyA⁺ARNs.

### Synthèse in vitro de l'ADN complémentaire (ADNc).

Les réactions de transcription reverse, au départ de 0,1 à 5 µg de polyA⁺ARNs, sont amorcées avec de l'oligo(dT)₁₂₋₁₈ (environ 1 µg; origine: BOEHRINGER). L'ADNc monobrin est ensuite transformé en molécule à double brin (ADNcdb) avec le même enzyme transcriptase reverse. Les préparations d'ADNcdb, généralement 1 µg, sont traitées avec la SI nucléase pour créer des extrémités franches. Les procédés sont bien connus de l'homme de métier et sont décrits en détail par T. MANIATIS et coll., loc.cit. L'ADNcdb est ensuite allongé par des extensions d'oligo(dC) selon le procédé décrit par L. VILLA-KOMAROFF et coll., (Proc.Natl.Acad.Sci.USA,75,(1978),3727-3731). Habituellement, on traite 100 ng d'ADNcdb par l'enzyme déoxynucléotidyl transférase terminale. En général, des extensions d'une longueur de 15 bases sont ajoutées aux extrémités 3' des molécules d'ADNcdb.

### Clonage de l'ADNcdb allongé dans le vecteur plasmidique pBR322.

L'ADN du plasmide pBR322 est linéarisé par l'enzyme Pstl et allongé par des extensions d'oligo(dG) selon la méthode décrite par R.M. LAWN et coll., (Nucleic Acids Res.9,(1981),6103-6114). L'ADNcdb allongé par les extensions d'oligo(dC) sont mélangés en proportions équimoléculaires avec l'ADN du pBR322 allongé par des extensions d'oligo(dG). D'habitude, 50 µg du mélange sont hybridés pour recirculariser le plasmide. Les conditions sont bien connues de l'homme du métier et sont décrites en détail par R.M. LAWN et coll., loc.cit. Le mélange d'hybridation est ensuite utilisé pour transformer des cellules compétentes de la souche MM294 de E. coli, par exemple, selon la méthode décrite par R.M. LAWN et coll., loc.cit. Plusieurs centaines de transformants sont obtenus par sélection par croissance sur milieu de tétracycline, la résistance à cet antibiotique ayant été apportée par le plasmide pBR322. Des transformants ont aussi été testés pour leur sensibilité à l'ampicilline. Ceux qui sont sensibles contiennent un plasmide chimérique puisque l'insertion de l'ADN étranger dans le vecteur inactive le gène de l'ampicilline.

### Criblage de la banque d'ADNc.

Les transformants E. coli sont criblés au moyen d'oligonucléotides synthétiques, marqués à leur extrémité 5' au ^{32p} et correspondant à un fragment du gène de l'apo A-I. La séquence des nucléotides de l'apo A - humaine est connue (voir P. CHEUNG et L. CHAN, loc.cit. et J.J. SEILHAMER et coll., loc.cit.); il est par conséquent pratique de synthétiser chimiquement par la méthode de N.D. SINHA et coll. (Nucleic Acids Res.12,(1984),4539-4557), une sonde d'oligonucléotides d'une longueur de 22 bases correspondant à l'extrémité 5' du gène. La séquence sélectionnée est la suivante: 5' - GCTGCGGTGCTGACCTTGGCCG - 3'. Avant d'être utilisé dans des expériences d'hybridation, l'oligonucléotide synthétique est phosphorylé à son extrémité 5' au moyen de la polynucléotide kinase de T4 (P - L Bicohemicals) et de (gamma-^{32p}]ATP. Les conditions de marquage et d'hybidation sont bien connues de l'homme du métier et sont décrites en détail par T. MANIATIS et coll., loc.cit. et par A. BOLLEN et coll., (DNA,2,(1983),255-264).

### Construction des plasmides d'expression.

Les méthodes pour la préparation de l'ADN, pour l'isolation de fragments d'ADN, de même que les conditions d'analyse par des enzymes de restriction et les conditions de ligation des fragments sont bien connues de l'homme du métier et sont décrites en détail par R.M. LAWN et coll., loc.cit. et par T. CABEZON et coll., loc.cit. et sont applicables ici.

La synthèse des fragments reliant le promoteur des vecteurs d'expression à l'extrémité 5' du gène sont décrites ci - dessous.

### Synthèse du fragment Ncol - Ball.

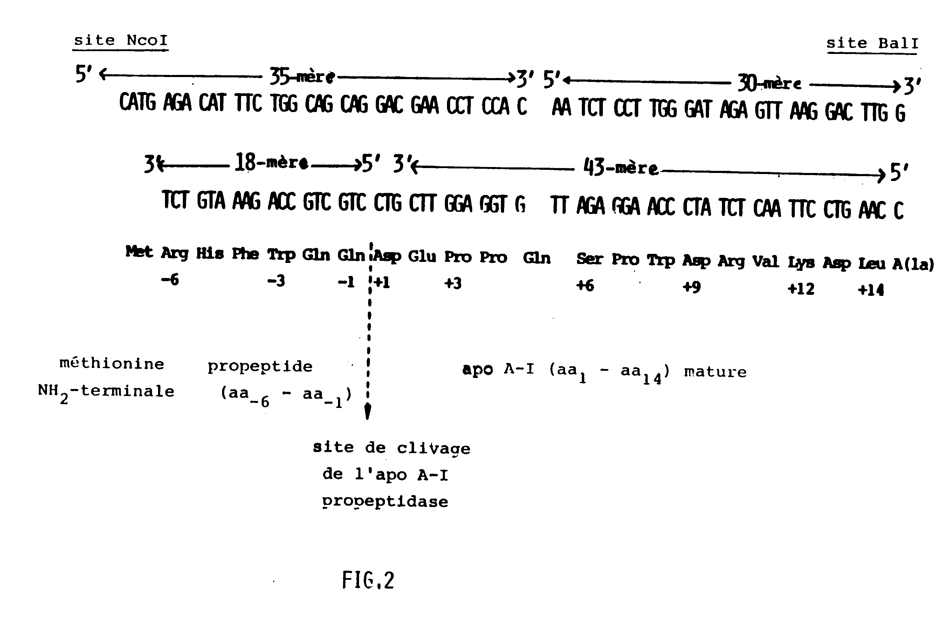

A la figure 2, on montre en détail les principes qui sont à la base de la conception des olgonucléotides 35 - mère, 30 - mère, 18 - mère et 43 - mère utilisés dans la synthèse du fragment Ncol - Ball de 65/61 pb. Les fragments synthétiques 30 - mère et 18 - mère sont phosphorylés à leurs extrémités 5' au moyen de la polynucléotide kinase de T4 (P - L Biochemicals). 1 µg de chaque oligonucléotide, y compris les 35 - mère et 43 - mère non phosphorylés sont hybridés pendant 3 minutes à 95 ° C dans 300 mM d'acétate de sodium (pH 7.0), puis laissés refroidir lentement à 4 ° C. Le mélange d'hybridation est utilisé tel quel dans le procédé de clonage pour la construction des plasmides d'expression.

### Séquençage de l'ADN

L'analyse de la séquence de l'ADN a été effectuée selon la méthode décrite par A.M. MAXAM et W. GILBERT (Proc.Natl.Acad.Sci.USA,74,(1977),560 - 564) et par F. SANGER et coll., (Proc.Nat!.Acad.Sci.USA,74,(1977),5463 - 5467).

### Analyse des protéines.

Des culots de cellules ayant une densité optique de 1 unité à 630 nm (1 OD₆₃₀) sont obtenus à différentes étapes pendant la fermentation de souches portant les plasmides d'expression de la proapo A-I humaine, pULB9291, pULB9292, pULB9296 et pULB9299 (transformés respectivement dans les souches AR58 et JM101 de E. coli et dans la souche 10S44c de levure). Chaque échantillon est remis en suspension dans un tampon 50 nm Tris - HCI, pH 6,8, contenant 2% de dodécylsulfate de sodium (DSS), 6 M d'urée, 10% de glycérol et 5% de 2-mercaptoéthanol, et est chauffé à l'ébullition pendant 3 minutes. Des échantillons sont soumis à une électrophorèse en gels de polyacrylamide (U.K. LAEMMLI, (Nature,227,(1970),680-685). Les protéines totales sont révélées par coloration au Bleu de Coomassie et la proapo A-1 humaine synthétisée est identifiée par reconnaissance immunologique après transfert électro - phorétique (voir A. BOLLEN et coll., loc.cit.).

### Construction de vecteurs recombinants pour l'expression de la proapo A-I humaine dans la bactérie, dans la levure et dans des cellules d'insectes infectées par baculovirus.

### 1. Clone d'ADNc pour la préproapo A-I humaine: pULB1609 (figure 1).

Plusieurs centaines de transformants, provenant du clonage de l'ADNcdb, correspondant au polyA⁺ARN de foie humain, dans le site Pstl de pBR322, sont criblés au moyen de la sonde synthétique apo A-I 22 - mère décrits ci-dessus. Un des clones donne un intense signal d'hybridation au cours de l'expérience. Ce clone, désigné par pULB1609, est isolé, et l'insert d'ADN présent dans le plasmide recombinant est caractérisé par analyse de la séquence de l'ADN. Sa longueur correspond à 878 paires de bases (pb); il code pour le polypeptide préproapo A- 1 complet. Comme le montre la figure 1, le fragment ADNc cloné porte des régions non codantes en 5' et en 3' (19 pb et 55 pb respectivement), la séquence de 54 pb codant pour le peptide précurseur (aa-24 jusque aa - 7), la séquence de 18 pb codant pour le propeptide (aa - jusque aa-1) et la séquence de 732 pb codant pour l'apo A-I mature (aa1 jusque aa243) et comprenent le codon de terminaison de la traduction. La séquence de la protéine, déduite de la séquence d'ADN, correspond parfaitement bien à la séquence des acides aminés trouvée pour la préproapo A-I à à partir de la protéine et à partir de clones d'ADNc isolés indépendamment (W.C. BARKER et coll., Comp.Biochem.Physiol.57B,(1977),309-315; demande de brevet japonaise No. 96998/86; P. CHEUNG et L. CHAN, loc.cit. et J.J. SEILHAMER et coll., loc.cit.).

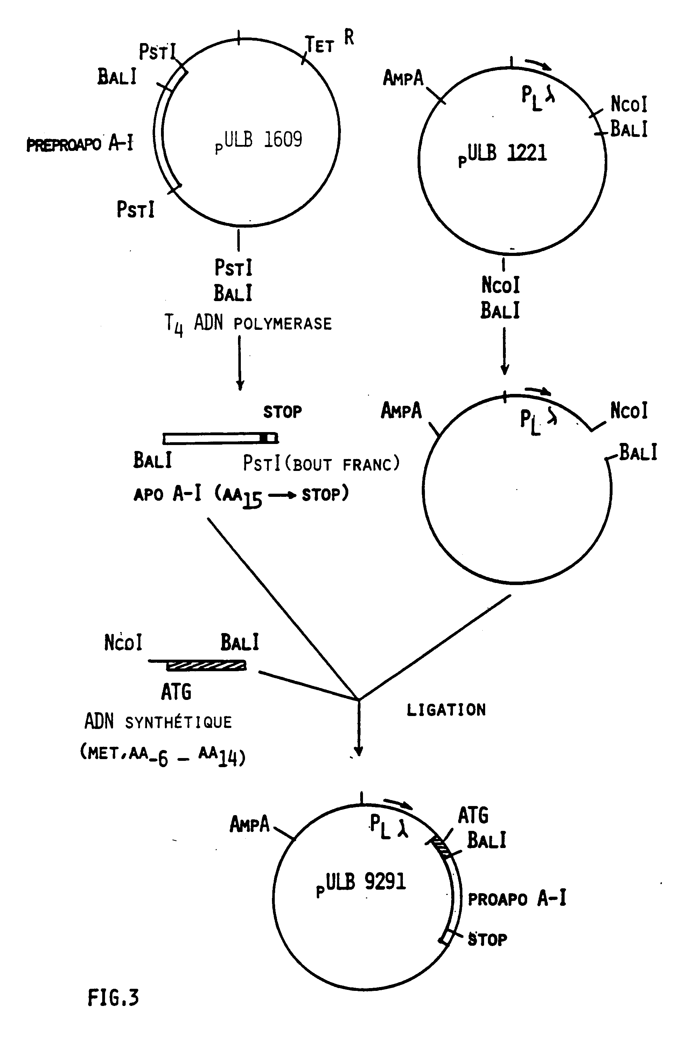

### 2. Construction d'un vecteur d'expression bactérien contenant la séquence de l'ADNc de la proapo A-I humaine: pULB9291 (figures 2 et 3).

pULB9291, un plasmide produisant la proapo A-1 humaine, est construit en plaçant un segment provenant du clone pULB1609 derrière le promoteur régulable P_{L} lambda (figure 3). La construction de ce plasmide d'expression requiert la synthèse de fragments d'ADN comprenant un site de restriction Ncol, un codon d'inititiation de la traduction et la séquence des nucléotides codant pour les acides aminés à l'extrémité aminée du gène de structure de la proapo A-1 humaine, jusqu'au premier site unique de restriction, Ball (figure 2).

Cet adapteur est synthétisé par des procédés chimiques (N.D. SINHA et coll., loc.cit.). On prépare quatre oligonucléotides synthétiques; après avoir été hybridés, ils codent pour la méthionine correspondant au codon ATG d'initiation de la traduction, pour les 6 acides aminés correspondant au propeptide et pour les 14 premiers acides aminés de l'apo A-I humaine mature (figure 2). L'adapteur synthétique a été conçu pour minimiser la formation de structures secondaires à l'extrémité 5' du gène. Pour réaliser cela, les codons sélectionnés pour coder les résidus d'acides aminés -6, -1, 1, 3, 4, 5, 6, 7, 10, 11 et 14 ne correspondent pas aux codons naturels que l'on observe dans l'ADNc du clone pULB1609.

L'adapteur synthétique décrit ci-dessus est utilisé pour réunir un fragment d'ADN de 744 pb, provenant de pULB1609, au promoteur P_{L} lambda dans le plasmide d'expression pULB1221.

La construction du vecteur d'expression pULB1221 est décrite dans la demande de brevet européen 186.643. Elle comporte trois étapes principales au départ du plasmide pCQV2. Le plasmide pCQV2 est décrit par C. QUEEN dans J.Mol.Appl.Genet.2,(1983)1 - 10 et est librement accessible.

Le choix de ce vecteur particulier n'est pas obligatoire: on peut utiliser tout autre vecteur possédant un promoteur et, en aval de celui - ci, un site Ncol approprié. Environ 0,1 µg des fragments synthétiques, tels que décrits ci-dessus, sont ligués, au moyen de l'ADN ligase de T4, à environ 1 µg du fragment Ball-Pstl de 744 pb, provenant de pULB1609, et à environ 1 µg du vecteur plasmidique pULB1221 coupé avec Ncol et Ball.

Avant de réaliser la ligation, le fragment Ball- Pstl de 744 pb a été traité par l'ADN polymérase de T4 de façon à transformer les extrémités protrudantes en 3' en extrémités franches. Le procédé est bien connu de l'homme du métier et est décrit en détail par T. MANIATIS et coll., loc.cit.

Après avoir été amplifiés dans les cellules compétentes de la souche AR58 de E. coli, les plasmides reconstruits sont caractérisé par analyse des sites de restriction et par la séquence de l'ADN des fragments synthétiques et des sites de jonction. Un plasmide recombinant, pULB9291, satisfait à tous les critères parce qu'il possède les fragments dans l'orientation et l'ordre corrects; il a été utilisé pour des études d'expression.

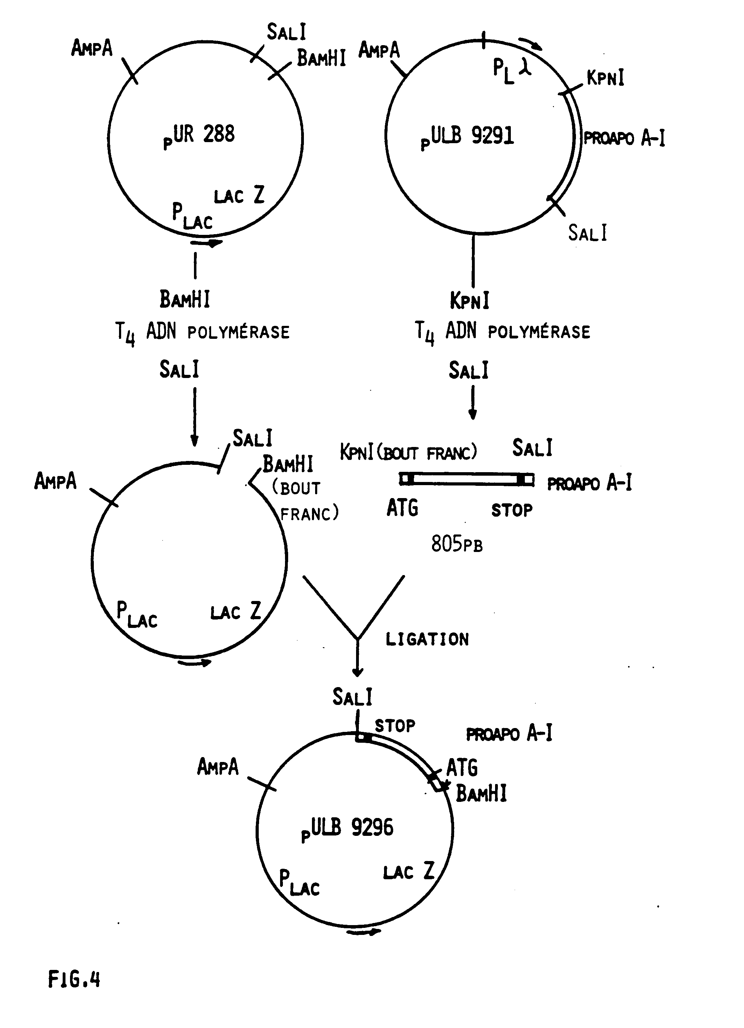

### 3. Construction d'un vecteur d'expression bactérien contenant les séquences fusionnées correspondant à la beta - galactosidase et à la proapa A - humaine: pULB9296 (figure 4).

Dans cette construction, la séquence d'ADN codant pour la proapo A-I humaine, est fusionnée dans la phase de lecture correcte, en aval de la séquence d'ADN de la beta - galactosidase. Le gène de la beta - galactosidase est présent dans le plasmide d'expression E. coli pUR288, librement accessible (U. RUTHER et B. MULLER- HILL, EMBO J.2,(1983),1791 1794), qui porte un promoteur lac efficacement inductible et des sites de restriction appropriés dans la séquence de la beta-galactosidase. Le plasmide recombinant approprié est construit comme montré à la figure 4. En premier lieu, l'ADN du plasmide pUR288 est, successivement, coupé par BamHl, traité par l'ADN polymérase de T4 et à nouveau coupé par Sall. En second lieu, un fragment d'ADN de 805 pb est isolé de pULB9291 successivement par digestion avec Kpnl, traitement par l'ADN polymérase de T4 et finalement digestion avec Sall. Les deux fragments sont ligués ensemble, dans des proportions molaires, au moyen de l'ADN ligase de T4 et le plasmide obtenu est utilisé pour transformer les cellules compétentes de la souche JM101 de E. coli, une souche largement et librement accessible (ATCC No. 33876). Les transformants sont vérifiés par analyse de restriction pour l'orientation correcte de la séquence de la proapo A-1 humaine par rapport au gène de la beta- galactosidase et pour la présence d'un site BamHl reconstitué à la jonction des deux séquences. Ceci indique que la séquence de la proapo A-I humaine est bien fusionnée en aval de la séquence d'ADN de la beta - galactosidase et dans la phase de lecture correcte. Un des transformants, contenant le plasmide pULB9296, satisfait à tous les critères et a été utilisé dans des essais d'expression.

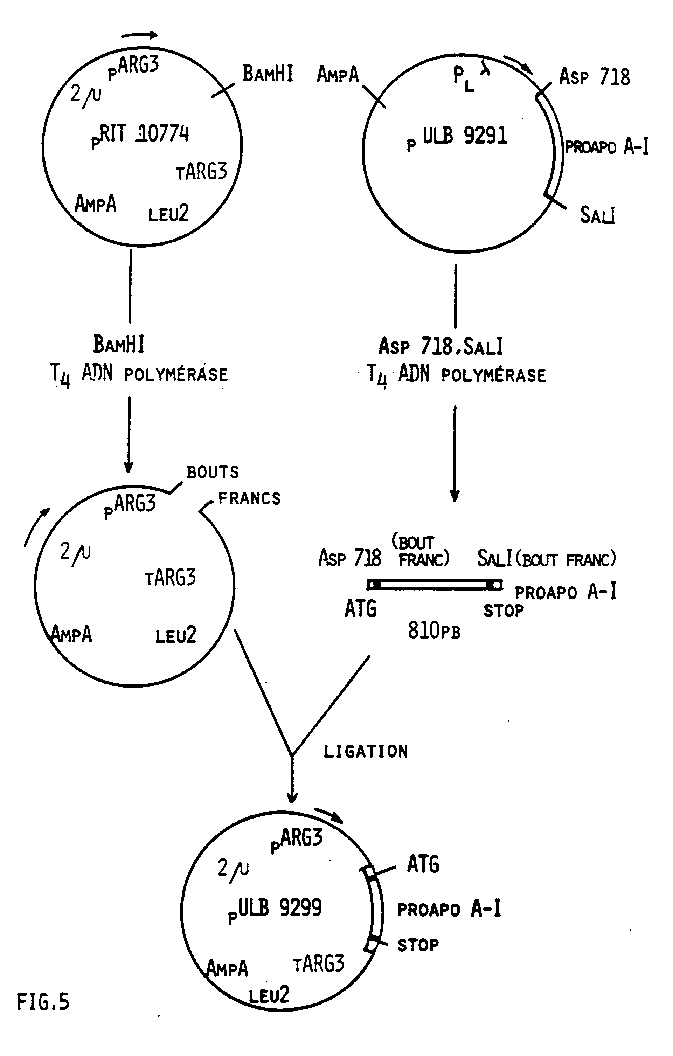

### 4. Construction d'un plasmide d'expression de levure porteur de la séquence de l'ADNc de la proapo A-I humaine: pULB9299 (figure 5).

Dans cette construction, la séquence de l'ADNc codant pour la proapo A-I humaine est clonée entre les signaux du promoteur et du terminateur portés par un plasmide d'expression de levure. Le vecteur d'expression de levure pRIT 10774 a été choisi pour cette expérience. La construction du plasmide d'expression pRIT 10774 est décrite dans la demande de brevet européen 151.102. On le construit, d'une part, à partir du plasmide pRIT 10749 déposé à l'ATCC sous le numéro d'accès 39133 conformément aux dispositions du Traité de Budapest, et d'autre part, à partir du vecteur-navette YEp13 pour E. coli - S. cerevisiae, décrit par J.R. BROACH et coll., dans Gene,8,(1979),121 - 133 et qui est accessible à l'ATCC sous le numéro d'accès 37115. Le vecteur pRIT 10774 peut se répliquer à la fois dans E. coli et dans la levure et porte le promoteur et le terminateur de la transcription de l'ornithine carbamoyl transférase (ARG3) séparés par un site uniqe de restriction BamHl convenant pour l'insertion d'un ADN étranger possédant son propre codon ATG d'initiation de la traduction. En outre, le vecteur porte les séquences 2µ, de la levure, des marqueurs métaboliques pour la sélection dans la levure et le marqueur de sélection AmpA pour faire la navette dans E. coli. Ce n'est pas le seul vecteur qui peut être utilisé pour l'expression de la proapo A-I humaine dans des levures: tout autre vecteur pour levure porteur de signaux de régulation peut être utilisé et conduira à des résultats qualitativement similaires. La construction illustrée à la figure 5 procède de la manière suivante. L'ADN du plasmide pRIT 10774 est linéarisé au moyen de l'enzyme BamHl et est traité par l'ADN polymérase de T4.

D'autre part, un fragment d'ADN de 810 pb est isolé de pULB9291 par digestion avec les enzymes Asp718 et Sall, suivie du traitement par l'ADN polymérase de T4. Ce fragment code pour la proapo A-I humaine et comporte le codon ATG d'initiation de la traduction. Les deux fragments, obtenus comme décrit ci-dessus, sont ligués ensemble, dans des proportions équimoléculaires, au moyen de l'ADN ligase de T4 et le mélange est utilisé pour transformer les cellules compétentes de E. coli MM294. Les transformants sont contrôlés par analyse de restriction pour vérifier l'orientation correcte de la séquence de l'ADN de la proapo A-I par rapport à la séquence du promoteur de l'ARG3. Un des transformants contient un plasmide recombinant, pULB9299, qui satisfait à cette condition. Le plasmide pULB9299 est amplifié dans E. coli et est utilisé pour transformer des sphéroplastes de la levure Saccharomyces cerevisiae, souche 10S44c (pep4 - 3, leu2 - 3, leu2 - 112); (T. CABEZON et coll., loc.cit.).

L'emploi de la souche Ic 1697d (ATCC No. 20631) conduirait à des résultats qualitativement similaires. Les transformants de levure ont ensuite été testés pour l'expression de la proapo A-I humaine.

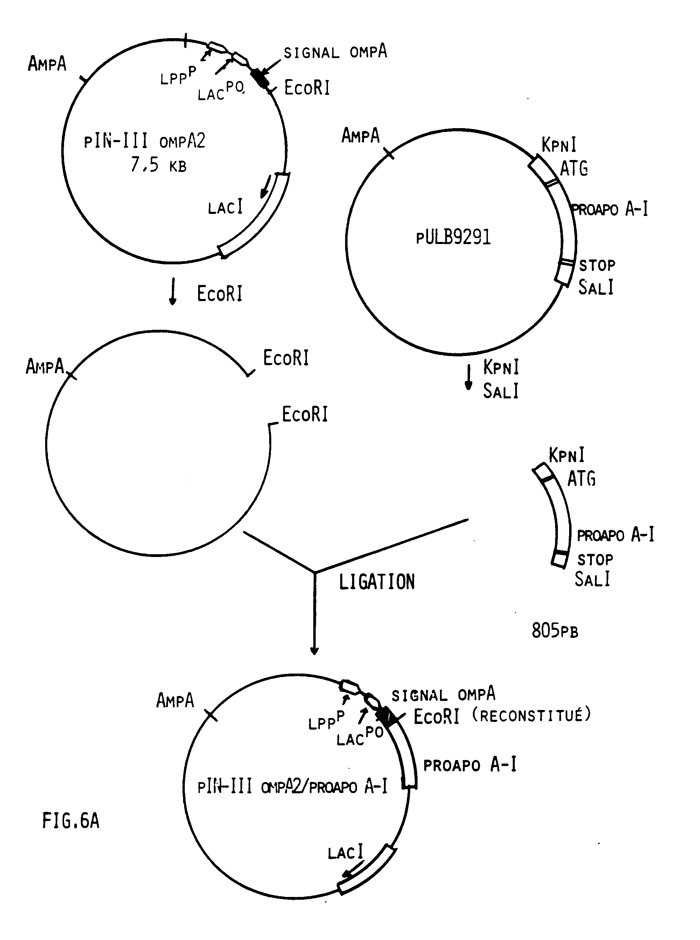

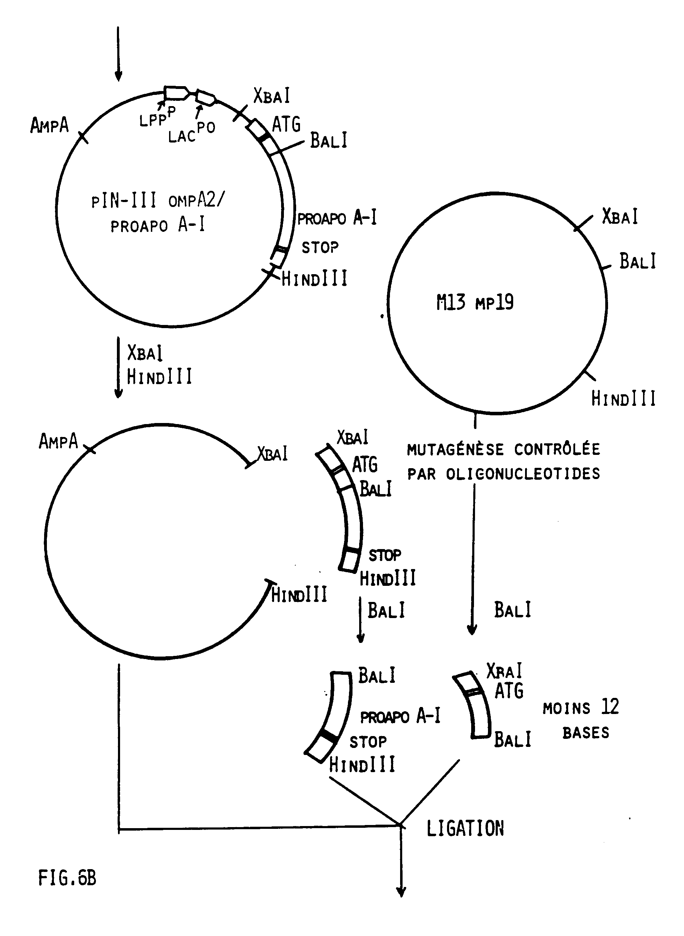

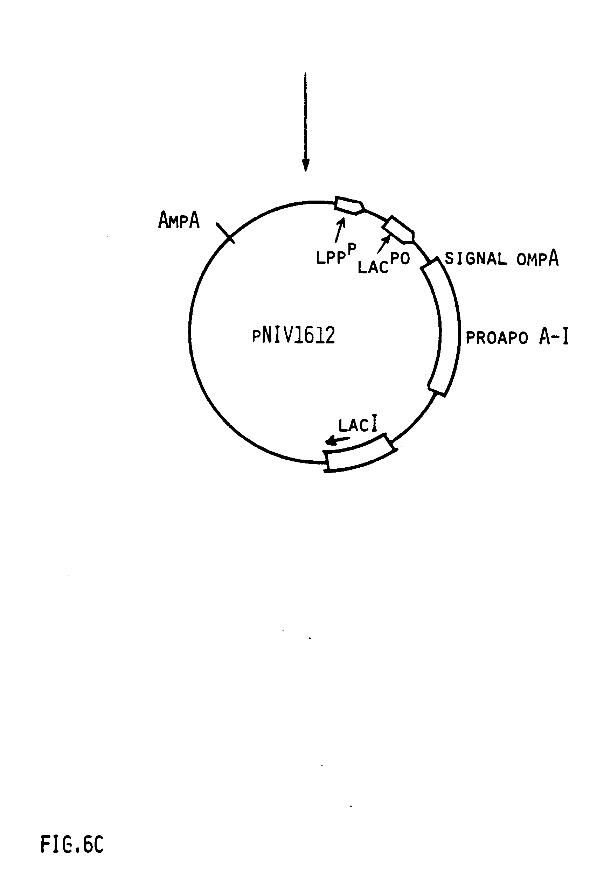

### 5. Construction d'un vecteur bactérien de sécrétion contenant la séquence de l'ADNc de la proapo A-I humaine: pNIV1612 (figures 6A, B et C).

Dans cette construction, la séquence d'ADN codant pour la proapo A-I humaine est fusionnée, dans la phase de lecture correcte, en aval de la séquence d'ADN du peptide signal de la protéine OmpA de E. coli. Le vecteur sécrétion pIN-III-ompA-2 2 (J. GHRAYEB et coll., EMBO J.3,(1984),2437-2442) a été choisi pour cet essai. Ce vecteur et sa souche hôte E. coli JA221 sont accessibles sur demande auprès du "Department of Biochemistry of the State University of New York", à Stony Brook.

Le vecteur de sécrétion porte un puissant promoteur Ipp (lipoprotéine), un fragment du promoteur du promoteur-opérateur lac, la séquence lacl du répresseur lac et des sites de restriction appropriés situés immédiatement après la séquence codant pour le peptide signal du gène ompA. Le plasmide recombinant approprié est construit comme montré aux figures 6A, B et C. En premier lieu, le vecteur de sécrétion pIN-III-ompA-2 est linéarisé avec EcoRI et traité par l'ADN polymérase de T4. En second lieu, un fragment d'ADN de 805 pb est excisé de pULB9291 par digestion, successivement avec les enzymes de restriction Kpnl et Sall, suivie du traitement par l'ADN polymérase de T4. Ce fragment code pour la proapo A-I humaine et contient le codon ATG d'initiation de la traduction. Les deux fragments obtenus comme décrit ci-dessus, sont ligués ensemble, en proportions équimolaires, au moyen de l'ADN ligase de T4, et le mélange est utilisé pour transformer les cellules compétentes de la souche JA221 de E. coli cultivées sur le milieu M9 (J.H. MILLER, dans "Experiments in Molecular Genetics", Cold Spring Harbor Laboratory, Cold Spring Harbor, New York, 1972, p.431) contenant 20 mg/l de tryptophane, 20 mg/l de leucine, 2 g/I de lactose et 50 mg/l d'ampicilline. Les transformants sont sélectionnés pour leur résistance à l'ampicilline et criblés avec un oligonucléotide synthétique 18-mère (le même que celui utilisé dans la synthèse de l'adapteur comme montré à la figure 2).

Les transformants sélectionnés sont contrôlés par analyse de restriction pour vérifier l'orientation correcte de la séquence d'ADN de la proapo A-I par rapport à la séquence du peptide signal porté par le vecteur de sécrétion (site EcoRI reconstitué à la jonction des deux séquences). Un des transformants porte un plasmide recombinant qui satisfait à cette condition. La séquence excédentaire provenant de la construction avec l'adapteur, soulignée dans la séquence nucléotidique suivante, 
5' GTA GCG GAG GCC GCT GAA TTC ATG AGA CAT TTC TGG 3'
Val Ala Gln Ala Ala Glu Phe Met Arg His Phe Trp
aa-6
 est éliminée par mutagénèse dirigée. A cet effet, on synthétise l'oligonucléotide 24-mère suivant 
peptide signal ---> <--- proapo A-I
5' GTA GCG GAG GCC AGA CAT TTC TGG 3'
Val Ala Gln Ala Arg His Phe Trp
 dépourvu des 12 bases excédentaires et on l'utilise pour éliminer la séquence excédentaire de 12 bases provenant de l'adapteur.

Pour la mutagénèse, on utilise le système de Amersham. Ce système est basé sur la méthode de F. ECKSTEIN et coll. (Nucleic Acids Res.13,(1985),8765-8785). La méthode donne un haut rendement en plages et l'efficacité la plus élevée dont on dispose: jusqu'à 95%.

En premier lieu, la région de l'ADN devant être mutagénisée est clonée dans un vecteur M13. A cet effet, le fragment d'ADN Xbal -Ball du plasmide recombinant pIN-III-ompA-2 portant le gène de la proapo A-1 est inséré dans le vecteur M13mp19 coupé par Xbal et Hindll. Le vecteur M13mp19 est disponible dans le commerce; on peut se le procurer auprès de Amersham (Buckinghamshire, Grande - Bretagne). Ensuite, l'oligonucléotide 24-mère mutagène est hybridé avec la matrice monocaténaire et allongé par le fragment de Klenow de l'ADN polymérase en présence de l'ADN ligase de T4, pour produire un mutant hétéroduplex.

L'élimination sélective du brin non mutant est rendue possible par l'incorporation d'un thionucléotide dans le brin mutant pendant la synthèse in vitro, et clivage par NCil du brin non phosphorothionaté. De tels clivages présentent des sites pour l'exonucléase III qui digère le brin non mutant. Le brin mutant est alors utilisé comme matrice pour reconstruire la molécule circulaire à double brin, créant ainsi une molécule mutante homoduplex. La mutagénèse est vérifiée par séquençage de la jonction entre le peptide signal et le début du gène de la proapo A-I. Le plasmide recombinant pNIV1612, est reconstruit en liguant le fragment Xbal- Hindlll du vecteur pIN- III -ompA-2 2 avec le fragment Xbal- Ball qui a été débarrassé des 12 bases excédentaires, et avec le fragment Ball- Hindlll du gène de la proapo A- I.

Les trois fragments sont ligués au moyen de l'ADN ligase de T4 et le mélange est utilisé pour transformer les cellules compétentes de E. coli JA221, comme décrit plus haut. Les transformants sont sélectionnés pour leur résistance à l'ampicilline et criblés avec l'oligonucléotide 18-mère cité ci-dessus. Un des transformants porte un plasmide recombinant pNIV1612. Dans la construction finale, la séquence codant pour le peptide signal de ompA précède la séquence complète de la proapo A-I sans le codon ATG (figures 6A, B et C). Les transformants E. coli sont ensuite testés pour l'expression de la proapo A-I humaine.

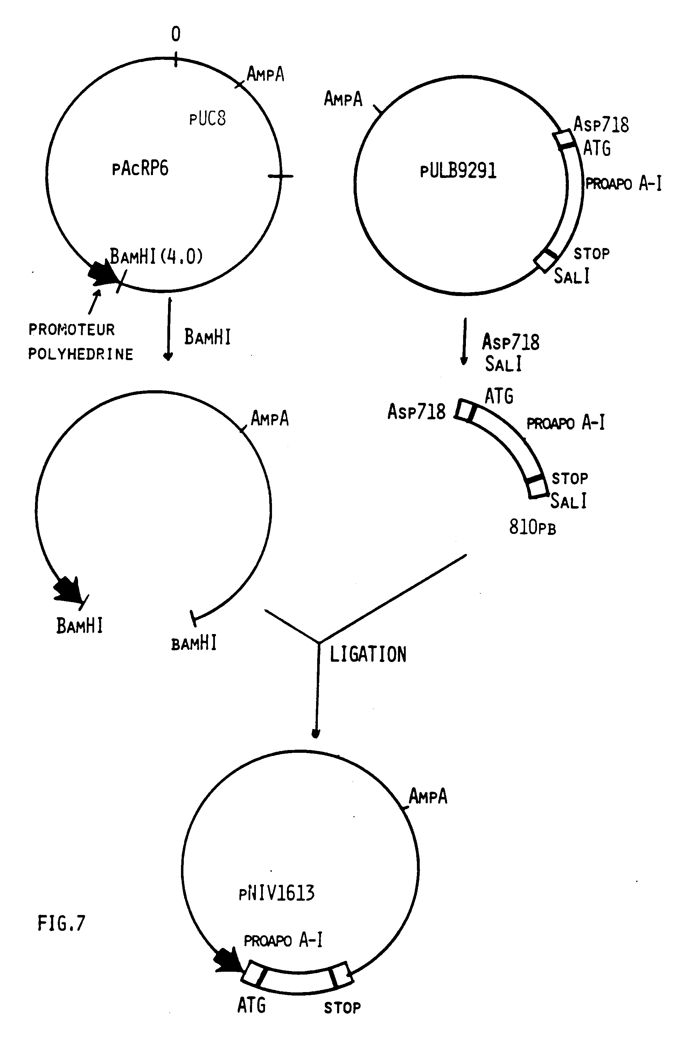

### 6. Construction d'un vecteur de transfert pour l'introduction de la séquence de l'ADNc de la proapo A-I humaine dans baculovirus: pNIV1613 (figure 7).

Le plasmide pNIV1613, portant la séquence d'ADN de la proapo A-I humaine, est construit en plaçant un segment provenant du clone pULB9291, en aval du promoteur du gène de la polyhédrine du baculovirus (figure 7). Dans ces essais, on a utilisé le vecteur de transfert pAcRP6 du Baculovirus (Y. MATSUURA et coll., J.Gen.Virol.67,(1986),1515-1529); il peut être obtenu auprès du "Department of Microbiology and Immunology" de l'Université d'Ottawa. Le plasmide porte le promoteur du gène de la polyhédrine jusqu'au nucléotide -7 dans la séquence leader en 5'; il lui manque le codon ATG de la polyhédrine et les 170 premiers nucléotides de la séquence codante de la polyhédrine. Un site BamHl approprié est localisé en aval du nucléotide -7. La construction illustrée à la figure 7 est réalisée de la façon suivante. L'ADN du plasmide pAcRP6 est linéarisé au moyen de BamHl. Par ailleurs, un fragment d'ADN de 810 pb est excisé de pULB9291 par digestion avec les enzymes de restriction Asp718 et Sall. Ce fragment code pour la proapo A-1 humaine et contient le codon ATG d'initiation de la traduction. Les deux fragments, en proportions équimolaires, sont traités ensemble par l'ADN polymérase de T4, ligués au moyen de l'ADN ligase de T4 et utilisés pour transformer les cellules compétentes de la souche AR58 de E. coli. Les transformants sont sélectionnés pour leur résistance à l'ampicilline, criblés au moyen d'un oligonucléotide synthétique 35 - mère marqué au 32p (voir la figure 2), correspondant à une partie de la séquence d'ADN de la proapo A-I, et contrôlés par analyse de restriction pour vérifier l'orientation correcte de la séquence d'ADN de la proapo A-I par rapport au promoteur de gène de la polyhédrine. Un des transformants porte un plasmide recombinant, pNIV1613, qui satisfait à cette condition; il a été utilisé dans des essais d'expression.

### 7. Production de la proapo A-I humaine par les microorganismes transformés.

Des cultures de 20 ml de la souche AR58 ou JM101 de E. coli transformées respectivement par pULB9291 et par pULB9296, sont cultivées en milieu riche supplémenté de 50 µg/ml d'ampicilline (bouillon de culture LB, voir T. MANIATIS et coll., loc.cit. pour les détails expérimentaux) jusqu'à ce que la densité optique atteigne 0,6 à 630 nm. Dans le cas de pULB9291, l'induction du promoteur P_{L} lambda est réalisée en portant les conditions initiales de croissance de la culture de 30 ° C à 42 C, de manière à inactiver le répresseur du promoteur P_{L} lambda (M. ROSENBERG et coll., Methods Enzymol.101,(1983),123-138). L'induction est poursuivie pendant 20 minutes.

Dans le cas de pULB9296, l'induction du promoteur lac est réalisée en ajoutant à la culture, incubée à 37 ° C, l'inducteur chimique IPTG (isopropyl - beta - D- thiogalactoside) jusqu'à atteindre une concentration finale de 1 mM (R. LORENZETTI et coll., loc.cit.). L'induction est poursuivie pendant 60 minutes.

D'autre part, des cultures de 20 ml de cellules de levure 10S44c, transformées par pULB9299, sont cultivées à 30 ° C en milieu de base azoté pour levure (Difco) supplémenté de glucose (1 %), jusqu'à ce que la densité optique atteigne 0,3 à 630 nm. Aucun inducteur n'est nécessaire pusque dans ce cas particulier l'expression est constitutive.

On prélève des échantillons de 1 ml des cultures ci-dessus et on les centrifuge à 15.000 g pendant 5 minutes. Les culots obtenus sont lysés comme suit dans du dodécylsulfate de sodium (DSS) bouillant. Les culots sont remis en suspension dans 50 µl de tampon d'échantillon contenant du DSS (50 mM de Tris-HCI, pH 6,8, 2% de DSS, 6 M d'urée, 5% de 2-mercaptoéthanol et 10% de glycérol) et chauffés à l'ébullition pendant 3 minutes à 100 ° C. Les extraits sont ensuite centrifugés à 15.000 g pendant 10 minutes. Les échantillons sont ainsi prêts pour l'analyse par électrophorèse sur gels de polyacrylamide à 15% ou 7,5%, en présence de DSS, dans des conditions dénaturantes (U.K. LAEMMLI, loc.cit.).

Après l'électrophorèse, les gels de polyacrylamide sont lavés brièvement avec 40 ml d'eau distillée et avec 40 ml de tampon phosphate de sodium 50 mM à pH 7,5. Les protéines sont ensuite transférées électriquement des gels sur des feuilles de nitrocellulose pendant 2 heures à 60 V et 1,5 A dans le même tampon phosphate. (T. CABEZON et coll., loc.cit.). Les feuilles de nitrocellulose sont saturées d'albumine (1 %) dans 50 mM de tampon phosphate de sodium à pH 7,5, puis elles sont incubées à la température ambiante pendant une nuit en présence de sérum de lapin anti-apo A-I humaine à une dilution de 1/500 (et d'anticorps anti-beta-galactosidase monoclonaux de souris dans le cas de pULB9296) dans le même tampon dépourvu d'albumine.

Les feuilles sont lavées 5 fois avec 40 ml du même tampon phosphate et sont ensuite incubées dans 40 ml de tampon phosphate contenant 10 µg/ml de sérum de chèvre anti - anticorps de lapin (ou anti - anticorps de souris) et marqué à la peroxydase. Après 4 heures d'incubation à la température ambiante, les feuilles sont à nouveau lavées 5 fois dans 40 ml de tampon phosphate et sont finalement révélées en ajoutant 50 ml d'une solution d'un substrat d'un substrat chromogène (10 mg de diaminobenzidine, 100 µl de peroxyde d'urée à 10% et 100 mM de Tris - HCI à pH 7,6).

Dans le cas de pULB9291 et de pULB9299, on trouve un produit unique réagissant avec les anticorps anti-apo A - humaine. Il a un poids moléculaire qui est conforme à celui d'un étalon d'apo A - naturelle. Dans le cas de pULB9296, on trouve à la dimension prévue pour la somme des polypeptides beta- galactosidase et proapo A-I (144 kDa), un polypeptide fusionné réagissant avec le sérum anti-apo A-I humaine et avec le sérum anti-beta-galactosidase. Ces dimensions ont été déterminées au préalable au moyen d'une courbe de calibrage basée sur la migration de poids moléculaires étalons placés sur les mêmes gels que les extraits cellulaires.

Dans une autre expérience, des cultures de 20 ml de la souche JA221 de E. coli transformée par pNIV1612 sont cultivées en milieu riche supplémenté de 50 µg/ml d'ampicilline (bouillon de culture LB, voir T. MANIATIS et coll., loc.cit.) jusqu'à ce que la densité optique atteigne 0,6 à 630 nm. L'induction du promoteur lac est effectuée en ajoutant à la culture, incubée à 37"C, l'inducteur chimique IPTG (isopropyl-beta-D-thiogalactoside) jusqu'à la concentration finale de 2 mM. L'induction et poursuivie pendant 60 minutes. On prélève des échantillons de 1 ml de ces cultures et on centrifuge à 15.000 g pendant 5 minutes. Les culots obtenus sont soumis à un choc osmotique léger en vue de libérer la fraction périplasmique (D. KOSHLAND et D. BOTSTEIN, Cell,20,(1980),749-760). La fraction libérée est remise en suspension dans le tampon d'échantillon contenant du DSS mentionné plus haut, mais dépourvu d'urée, elle est portée à l'ébullition, centrifugée et soumise à une électrophorèse sur gel de polyacrylamide à 12,5% en présence de DSS, suivie d'une immunodétection après transfert électrophorétique. Une fraction de la proapo A-I synthétisée est retrouvée dans la cellule et non pas dans le périplasme. Ceci est dû soit au fait que de la proapo A-I n'est pas sécrétée, soit à ce que l'efficacité du choc osmotique n'est pas optimale. La fraction principale de la proapo A-I se retrouve libérée dans le milieu après le choc osmotique, indiquant ainsi que la protéine est bien sécrétée par les cellules.

### 8. Production de la proapo A-I humaine par des cellules d'insectes infectées par baculovirus.

Le plasmide recombinant pNIV1613 a été utilisé conjointement avec la souche sauvage de baculovirus pour coinfecter des cellules de Spodoptera frugiperda en culture. Le tri des virus recombinants dépourvus de polyhédrine fournit des plages recombinantes. Le virus recombinant, purifié à partir d'une plage, est utilisé pour infecter des cellules d'insectes. Le procédé est bien connu de l'homme du métier et est décrit en détail par M.D. SUMMERS et G.E. SMITH dans "A Manual of Methods for Baculovirus Vectors and Insect cell culture Procedures, Texas University, College Station,(1987)". Le virus recombinant a été testé pour la production de proapo A- 1, immunologiquement après transfert électrophorétique et par électro - phorèse sur gel de polyacrylamide à 12,5% en présence de DSS, après lyse des cellules au moyen du tampon RIPA [0,05 M de tampon Tris - HCI, pH 7,2, contenant 0,15 M de NaCI, 1 % de Triton X100, 0,1% de DSS, 0,1% de désoxycholate de sodium et 1 mM de fluorure de phénylméthylsulfonyle (FPMS)] et traitement par du DSS bouillant. Un seul produit réagissant avec les anticorps anti-apo A-I humaine a été trouvé. Il a un poids moléculaire qui concorde avec celui d'un étalon d'apo A-I naturelle et la protéine exprimée représente le constituant majeur des protéines totales. La concentration en proapo A-I par litre de culture, mesurée par immunodiffusion radiale simple (G. MANCINI et coll., Immunochem.2,(1965),235-254) est estimée à environ 100 mg.

### 9. Production cytoplasmique de la proapo A-I humaine dans E. coli. Utilisation d'un milieu minimal défini.

Le plasmide pULB9292 a été utilisé pour la production cytoplasmique de proapo A-I humaine par E. coli dans un milieu minimal. pULB9292 est construit en échangeant le fragment EcoRI- Ncol du plasmide pULB9291, codant pour le promoteur P_{L} lambda, avec le même fragment EcoRI- Ncol du plasmide pOTS (M. ROSENBERG et coll., Methods Enzymol.101,(1983),123 138). Le fragment EcoRl Ncol du vecteur pOTS (G. DEVARE et coll., Cell,36,(1984),43-49) contient aussi le promoteur P_{L}, efficace et régulable, du phage lambda. On fait croître sur un milieu minimal défini des cultures de 20 ml de la souche AR58 de E. coli transformée avec pULB9292. La composition du milieu minimal est (par litre): 3 g de Na₂HP0₄.2H₂0, 3 g de KH₂P0₄, 0,5 g de NaCI, 1 g de NH₄CI, 1,37 mM de MgS0₄.7H₂0, 29,5 µM de FeCIa.6H₂O, 236 µM de MnS0₄.H₂0, 10 g de glucose, 1 mg de vitamine B1, 50 mg d'ampicilline, bouillon de culture LB 1/20 (v/v). Les cellules sont cultivées dans ce milieu minimal jusqu'à ce que la densité optique atteigne 0,5 à 630 nm. L'induction du promoteur P_{L} lambda est effectuée en portant les conditions initiales de croissance de la culture de 30 ° C à 42 ° C, de manière à inactiver le répresseur du promoteur P_{L} lambda (M. ROSENBERG et coll., loc.cit.). L'induction est poursuivie pendant 60 minutes. On prélève des échantillons de 1 ml des cultures et on les fait passer dans la presse de French ou on les centrifuge à 15.000 g pendant 5 minutes. L'extrait cellulaire total obtenu ou le culot sont traités par du DSS à l'ébullition comme décrit au paragraphe 7. Après électrophorèse et transfert électrophorétique, on trouve un seul produit qui réagit avec les anticorps anti-Apo A-I humaine. Il a un poids moléculaire qui concorde avec celui d'un étalon d'apo A-I 1 naturelle. Le taux de proapolipoprotéine A-I exprimé dans le milieu minimal défini représente 13,5% des protéines totales, soit une concentration estimée de proapo A-I d'environ 270 mg par litre de culture.

### 10. Isolement, purification et caractérisation de la proapo A-I humaine produite par les microorganismes transformés.

### 10.1 Isolement et purification.

On centrifuge les extraits bruts de la proapo A-I recombinante à 4.000 g pendant 15 minutes et on écarte le culot. On centrifuge le surnageant à 100.000 g pendant deux heures. On remet le culot obtenu en suspension dans un volume minimum d'un tampon (TEN100) composé de 20 mM de Tris-HCI, pH 7,5; 1 mM d'EDTA, 100 mM de NaCI; 1,75 µg/ml de FPMS et 100 µg/ml de merthiolate de sodium (sel de sodium de l'acide éthylmercuri-thiosalicylique) et les volumes de la suspension et du surnageant sont ajustés séparément au volume initial de l'extrait au moyen du même tampon. Ensuite, la protéine est précipitée à partir des deux suspensions en utilisant des concentrations croissantes d'alcool ispropylique. On détermine par immunodiffusion radiale (G. MANCINI et coll., loc.cit.), en utilisant un étalon commercial d'apo A-I, la fraction de protéine précipitée de chaque suspension qui contient la majeure partie de l'immunoréactivité correspondant à l'apo A-1 humaine. Chaque fraction ainsi obtenue est davantage purifiée par chromatographie sur une colonne de Sephacryl S200, en utilisant le même tampon comme éluant. On recueille des fractions de 0,9 ml et on détermine par immunodiffusion radiale, dans chaque fraction, la quantité de protéine totale ayant une immunoréactivité correspondant à celle de l'apo A-1. Le poids moléculaire de la protéine éluée dans les fractions est déterminé par calibrage de la colonne avec des étalons de poids moléculaires connus tels que l'aldolase, l'albumine de sérum de bovin, l'ovalbumine, le chymotrypsinogène et le cytochrome C, dans des conditions identiques. La pureté de la proapo A-I par mg de protéine totale dans les fractions principales contenant la proapo A-I recombinante, exprimée en mg de protéine ayant la même immunoréactivité qu'un étalon commercial d'apo A-I, est estimée à 95%.

### 10.2. Caractérisation.

### 10.2.1. Propriétés physiques de la proapo A-I recombinante.

Lorsqu'on soumet la proapo A-I recombinante purifiée et isolée du surnageant et du culot de la centrifugation à 100.000 g, à une focalisation isoélectrique selon le procédé de N. CATSIMPOOLAS (Anal.Biochem.26,(1969),54-62) et en appliquant un gradient autogénéré de pH 4 à pH 6, on obtient une bande unique ayant un point isoélectrique estimé à 4,95. L'apo A-I plasmatique humaine apparaît légèrement plus acide et a un point isoélectrique estimé à 4,75. Cette différence de 0,2 unité de pH entre les valeurs des points isoélectriques de la proapo A-I recombinante et de l'apo A-I plasmatique est en bon accord avec la différence connue entre les valeurs des points isoélectriques de l'apo A-I plasmatique et de la proapo A-I plasmatique, qui a été rapportée comme étant égale à 0,17 (G.L. MILLS et coll., Lab.Tech.Biochem.Mol.Biol.14,(1984),244 - 245).

En ce qui concerne le poids moléculaire, les proapo A-I recombinantes du culot et du surnageant consistent toutes deux en une chaîne polypeptidique unique ayant des poids moléculaires identiques égaux à 29,9 ± 1,4 kDa. L'apo A- plasmatique humaine apparaît légèrement plus petite, ayant un poids moléculaire égal à 29,3 ± 1,3 kDa.

### 10.2.2. Etablissement d'une carte de peptides de la proapo A-1 recombinante au moyen de BNPS-scatole.

Le clivage chimique a été effectué au moyen de 3-bromo-3-méthyl-2-[(2-nitrophényl)thio]-3H - indole (BNPS - scatole) selon le procédé de A. FONTANA (Methods Enzymol.25,(1972),419 - 423). On dissout 5 à 10 µg des préparations de protéine purifiée dans 100 µl d'une solution à 0,15% (v/v) de phénol dans une solution aqueuse à 50% (v/v) d'acide acétique. Ensuite, on ajoute 50 µl d'une solution de 4,8 mg de BNPS-scatole par ml d'acide acétique glacial et on incube à 25°C pendant 72 heures. Ensuite, on ajoute 50 µl de 2-mercaptoéthanol et on incube une seconde fois pendant 5 heures à 37 ° C. Les échantillons sont évaporés, redissous dans 100 µl d'eau et extraits 3 fois par 200 µl d'acétate d'éthyle. Les phases organiques sont écartées et les phases aqueuses sont lyophilisées et analysées par électrophorèse sur gel de polyacrylamide - DSS.

Dans le cas d'un clivage chimique par le BNPS-scatole, le nombre et la dimension des fragments provenant d'apo A-I peuvent être plus ou moins prédits étant donné que, dans les conditions expérimen - tales utilisées, le BNPS-scatole coupe sélectivement après les résidus de tryptophane. En supposant une efficacité de 100% à chaque site de clivage, le fragment le plus grand auquel on peut s'attendre est un fragment C-terminal de 15,4 kDa. Les poids moléculaires des fragments restants sont compris entre 0,5 et 5,3 kDa et sont par conséquent trop petits pour être détectés.

Dans le cas d'un clivage incomplet, le fragment de 15,4 kDa sera "allongé" dans la direction de l'extrémité N-terminale, formant des fragments de 20,7 kDa, 23,1 kDa et 27,6 kDa respectivement. Ces prévisions sont très bien réalisées pour l'apo A-I plasmatique humaine de même que pour les différentes préparations purifiées de proapo A-I recombinante.

## Revendications (Revendications pour l'(les) Etat(s) contractant(s) suivant(s) : suivants : AT, BE, CH, DE, FR, GB, GR, IT, LI, LU, NL, SE)

1. Séquence d'ADN recombinante qui code pour la proapolipoprotéine A-1 humaine et capable de réduire ou prévenir la formation de structures en épingle, caractérisée en ce qu'elle comprend (a) un fragment d'ADN synthétique ayant la séquence (un seul brin indiqué): 5' - ATGAGACATTTCTGGCAGCAGGACGAACCTCCACAATCTCCTTGGGATAGAGTTAAGGACTTG - 3' codant pour les acides aminés - -6 à +14 de la proapolipoprotéine A-I humaine et comprenant un codon supplémentaire ATG d'initiation de la traduction et des codons modifiés pour les résidus des acides aminés -6, -1, + 1, +3, +4, +5, +6, +7, + 10, +11 et +14 et (b) en aval du fragment d'ADN synthétique, la séquence d'ADN naturelle codant pour les acides aminés +15 à +243 de la proapolipoprotéine A-I humaine.

2. Vecteur de clonage réplicable, caractérisé en ce qu'il contient la séquence d'ADN recombinante selon la revendication 1.

3. Vecteur d'expression, caractérisé en ce qu'il contient la séquence d'ADN recombinante selon la revendication 1 liée de manière opérationnelle à une séquence d'ADN régulatrice qui règle l'expression de ladite séquence d'ADN recombinante.

4. Vecteur d'expression selon la revendication 3, caractérisé en ce que la séquence d'ADN régulatrice comprend la région du promoteur P_{L} du phage lambda.

5. Vecteur d'expression selon la revendication 3, caractérisé en ce que la séquence d'ADN recombinante est fusionnée en aval de la séquence d'ADN de la beta-galactosidase et en ce que la séquence d'ADN régulatrice comprend la région du promoteur lac de E. coli.

6. Vecteur d'expression selon la revendication 3, caractérisé en ce que la séquence d'ADN régulatrice comprend les régions du promoteur et du terminateur de la transcription de l'ARG3 de la levure.

7. Vecteur d'expression selon la revendication 3, caractérisé en ce que la séquence d'ADN recombinante dépourvue du codon ATG est fusionnée en aval de la séquence d'ADN du peptide signal de la protéine OmpA de E. coli et en ce que la séquence d'ADN régulatrice comprend le promoteur Ipp, le promoteur-opérateur lac et la séquence lac 1 du répresseur lac.

8. Vecteur d'expression selon la revendication 3, caractérisé en ce que la séquence d'ADN régulatrice comprend la région du promoteur du gène de la polyhédrine de baculovirus.

9. Culture de cellules ou microorganisme transformés avec un vecteur d'expression selon l'une quelcon - que des revendications 3 à 8.

10. Procédé de production de la proapolipoprotéine A - humaine, caractérisé en ce qu'on cultive dans des conditions de culture appropriées une cellule ou un microorganisme transformé selon la revendication 9, et en ce qu'on recueille la proapolipoprotéine A-I humaine ainsi produite.

## Revendications (Revendications pour l'(les) Etat(s) contractant(s) suivant(s) : suivant : ES)

1. Procédé de préparation d'une séquence d'ADN recombinante qui code pour la proapolipoprotéine A-I humaine et capable de réduire ou prévenir la formation de structures en épingle, caractérisé en ce qu'on ligue la séquence d'ADN naturelle codant pour les acides aminés +15 à +243 de la proapolipoprotéine A-I humaine en aval d'un fragment d'ADN synthétique ayant la séquence (un seul brin indiqué): 5' - ATGAGACATTTCTGGCAGCAGGACGAACCTCCACAATCTCCTTGGGATAGAGTTAAGGACTTG - 3' codant pour les acides aminés - -6 à +14 de la proapolipoprotéine A-I humaine et comprenant un codon supplémentaire ATG d'initiation de la traduction et des codons modifiés pour les résidus des acides aminés -6, -1, +1, +3, +4, +5, +6, +7, +10, + 11 et + 14.

2. Procédé de préparation d'un vecteur de clonage réplicable, caractérisé en ce qu'on introduit la séquence d'ADN recombinante préparée selon le procédé de la revendication 1 dans un vecteur approprié.

3. Procédé de préparation d'un vecteur d'expression, caractérisé en ce qu'on ligue de manière opérationnelle la séquence d'ADN recombinante préparée selon le procédé de la revendication 1 à une séquence d'ADN régulatrice qui règle l'expression de ladite séquence d'ADN recombinante.

4. Procédé selon la revendication 3, caractérisé en ce que la séquence d'ADN régulatrice comprend la région du promoteur P_{L} du phage lambda.

5. Procédé selon la revendication 3, caractérisé en ce que la séquence d'ADN recombinante est fusionnée en aval de la séquence d'ADN de la beta-galactosidase et en ce que la séquence d'ADN régulatrice comprend la région du promoteur lac de E. coli.

6. Procédé selon la revendication 3, caractérisé en ce que la séquence d'ADN régulatrice comprend les régions du promoteur et du terminateur de la transcription de l'ARG3 de la levure.

7. Procédé selon la revendication 3, caractérisé en ce que la séquence d'ADN recombinante dépourvue du codon ATG est fusionnée en aval de la séquence d'ADN du peptide signal de la protéine OmpA de E. coli et en ce que la séquence d'ADN régulatrice comprend le promoteur Ipp, le promoteur-opérateur lac et la séquence lac 1 du répresseur lac.

8. Procédé selon la revendication 3, caractérisé en ce que la séquence d'ADN régulatrice comprend la région du promoteur du gène de la polyhédrine de baculovirus.

9. Procédé de préparation d'une cellule ou d'un microorganisme, caractérisé en ce qu'on transforme une cellule ou un microorganisme avec un vecteur d'expression préparé selon un procédé tel que défini dans l'une quelconque des revendications 3 à 8.

10. Culture de cellules ou microorganisme transformés avec un vecteur d'expression préparé selon un procédé tel que défini dans l'une quelconque des revendications 3 à 8.

11. Procédé de production de la proapolipoprotéine A-I humaine, caractérisé en ce qu'on cultive dans des conditions de culture appropriées une cellule ou un microorganisme transformé selon la revendication 10, et en ce qu'on recueille la proapolipoprotéine A-I humaine ainsi produite.

## Claims (Claims for the following Contracting State(s) : AT, BE, CH, DE, FR, GB, GR, IT, LI, LU, NL, SE)

1. Recombinant DNA sequence coding for human proapolipoprotein A-I and capable of reducing or preventing the formation of hairpins, characterised in that it comprises (a) a synthetic DNA fragment having the sequence (only one strand shown): 5' - ATGAGACATTTCTGGCAGCAGGACGAACCTCCACAATCTCCTTGGGATAGAGTTAAGGACTTG - 3' coding for the amino acids - -6 to + 14 of human proapolipoprotein A-I and comprising an added ATG translation initiation codon and modified codons for the amino acid residues - 6, - -1, + 1, + 3, + 4, + 5, + 6, + 7, + 10, + 11 and + 14 and (b) downstream from the synthetic DNA fragment, the natural DNA sequence coding for the amino-acids + 15 to +243 of human proapolipoprotein A-I.

2. Replicable cloning vector, characterised in that it contains a recombinant DNA sequence according to claim 1.

3. Expression vector, characterised in that it contains a recombinant DNA sequence according to claim 1 operably linked to a regulatory DNA sequence which regulates the expression of said recombinant DNA sequence.

4. Expression vector according to claim 3, characterised in that the regulatory DNA sequence comprises the phage lambda P_{L} promoter region.

5. Expression vector according to claim 3, characterised in that the recombinant DNA sequence is fused downstream from the beta - galactosidase DNA sequence and in that the regulatory DNA sequence comprises the E. coli lac promoter region.

6. Expression vector according to claim 3, characterised in that the regulatory DNA sequence comprises the yeast ARG3 promoter and transcription terminator regions.

7. Expression vector according to claim 3, characterised in that the recombinant DNA sequence lacking the ATG codon is fused downstream from the DNA sequence of the E. coli OmpA protein signal peptide and in that the regulatory DNA sequence comprises the Ipp promoter, the lac promoter- operator and the lac I sequence of the lac repressor.

8. Expression vector according to claim 3, characterised in that the regulatory DNA sequence comprises the baculovirus polyhedrin gene promoter region.

9. Cell culture or microorganism transformed with an expression vector as claimed in any of claims 3 to 8.

10. Process for producing human proapolipoprotein A-I characterised in that a transformed cell or microorganism according to claim 9 is cultured under appropriate cultivation conditions, and in that the human proapolipoprotein A -I so produced is recovered.

## Claims (Claims for the following Contracting State(s) : ES)

1. Process for the preparation of a recombinant DNA sequence coding for human proapolipoprotein A-I and capable of reducing or preventing the formation of hairpins, characterised in that the natural DNA sequence coding for the amino-acids +15 to +243 of human proapolipoprotein A-I is ligated downstream from a synthetic DNA fragment having the sequence (only one strand shown): 5' - ATGAGACATTTCTGGCAGCAGGACGAACCTCCACAATCTCCTTGGGATAGAGTTAAGGACTTG - 3' coding for the amino acids - -6 to +14 of human proapolipoprotein A-I and comprising an added ATG translation initiation codon and modified codons for the amino acid residues - 6, - -1, + 1, + 3, + 4, + 5, +6, +7, +10, +11 and +14.

2. Process for the preparation of a replicable cloning vector, characterised in that a recombinant DNA sequence prepared according to the process of claim 1 is introduced in an appropriate vector.

3. Process for the preparation of an expression vector characterised in that a recombinant DNA sequence prepared according to the process of claim 1 is operably ligated to a regulatory DNA sequence which regulates the expression of said recombinant DNA sequence.

4. Process according to claim 3, characterised in that the regulatory DNA sequence comprises the phage lambda P_{L} promoter region.

5. Process according to claim 3, characterised in that the recombinant DNA sequence is fused down - stream from the beta-galactosidase DNA sequence and in that the regulatory DNA sequence comprises the E. coli lac promoter region.

6. Process according to claim 3, characterised in that the regulatory DNA sequence comprises the yeast ARG3 promoter and transcription terminator regions.

7. Process according to claim 3, characterised in that the recombinant DNA sequence lacking the ATG codon is fused downstream from the DNA sequence of the E. coli OmpA protein signal peptide and in that the regulatory DNA sequence comprises the Ipp promoter, the lac promoter - operator and the lac I sequence of the lac repressor.

8. Process according to claim 3, characterised in that the regulatory DNA sequence comprises the baculovirus polyhedrin gene promoter region.

9. Process for the preparation of a cell or a microorganism, characterised in that a cell or a microorganism is transformed with an expression vector prepared according to a process as defined in any of claims 3 to 8.

10. Cell culture or microorganism transformed with an expression vector prepared according to a process as defined in any of claims 3 to 8.

11. Process for producing human proapolipoprotein A-I, characterised in that a transformed cell or microorganism according to claim 10 is cultured under appropriate cultivation conditions, and in that the human proapolipoprotein A-I so produced is recovered.

## Patentansprüche (Patentansprüche für folgende(n) Vertragsstaat(en) : AT, BE, CH, DE, FR, GB, GR, IT, LI, LU, NL, SE)

1. Rekombinante DNA-Sequenz, die für das menschliche Proapolipoprotein A-I codiert und die Bildung von Haarnadel - Strukturen reduzieren oder verhindern kann, dadurch gekennzeichnet, daß sie umfaßt (a) ein synthetisches DNA-fragment mit der Sequenz (nur ein Strang dargestellt): 5' - ATGAGACATTTCTGGCAGCAGGACGAACCTCCACAATCTCCTTGGGATAGAGTTAAGGACTTG - 3' das für die Aminosäuren -6 bis +14 des menschlichen Proapolipoproteins A-I codiert und ein zusätzliches ATG-Codon zur Initiation der Translation und modifizierte Codons für die Reste der Aminosäuren - 6, - -1, + 1, + 3, + 4, + 5, + 6, +7, + 10, + 11 and + 14 umfaßt und (b) stromabwärts des Synthetischen DNA-Fragments die natürliche DNA-Sequenz, die für die Aminosäuren +15 bis +243 des menschlichen Proapolipoproteins A-I codiert.

2. Replizierbarer Klonierungsvektor, dadurch gekennzeichnet, daß er die rekombinante DNA-Sequenz nach Anspruch 1 enthält.

3. Expressionsvektor, dadurch gekennzeichnet, daß er die rekombinante DNA-Sequenz nach Anspruch 1 enthält, die auf operative Weise mit einer regulatorischen DNA-Sequenz, die die Expression der rekombinanten DNA-Sequenz steuert, verbunden ist.

4. Expressionsvektor nach Anspruch 3, dadurch gekennzeichnet, daß die regulatorische DNA-Sequenz den Bereich des Promotors P_{L} des Phagen lambda umfaßt.

5. Expressionsvektor nach Anspruch 3, dadurch gekennzeichnet, daß die rekombinante DNA-Sequenz stromabwärts mit der DNA-Sequenz der Beta-Galactosidase fusioniert ist und daß die regulatorische DNA-Sequenz den Bereich des Promotors lac von E. coli umfaßt.

6. Expressionsvektor nach Anspruch 3, dadurch gekennzeichnet, daß die regulatorische DNA-Sequenz die Bereiche des Promotors und des Terminators der Transkription von ARG3 der Hefe umfaßt.

7. Expressionsvektor nach Anspruch 3, dadurch gekennzeichnet, daß die rekombinante DNA-Sequenz ohne ATG - Codon stromabwärts von der DNA - Sequenz des Signalpeptids des OmpA - Proteins von E. coli fusioniert ist und daß die regulatorische DNA-Sequenz den Promotor Ipp, den Promotor-Operator lac und die Sequenz lac I des Repressors lac umfaßt.

8. Expressionsvektor nach Anspruch 3, dadurch gekennzeichnet, daß die regulatorische DNA-Sequenz den Bereich des Promotors des Polyhedrin - Gens des Baculovirus umfaßt.

9. Zellkultur oder Mikroorganismus, transformiert mit einem Expressionsvektor nach einem der Ansprüche 3 bis 8.

10. Verfahren zur Herstellung des menschlichen Proapolipoproteins A-I, dadurch gekennzeichnet, daß man unter geeigneten Kulturbedingungen eine transformierte Zelle oder Mikroorganismus gemäß Anspruch 9 kultiviert und, daß man das so hergestellte menschliche Proapolipoprotein A-I gewinnt.

## Patentansprüche (Patentansprüche für folgende(n) Vertragsstaat(en) : ES)

1. Verfahren zur Herstellung einer rekombinanten DNA-Sequenz, die für das menschliche Proapolipoprotein A- codiert und die Bildung von Haarnadel - Strukturen reduzieren oder verhindern kann, dadurch gekennzeichnet, daß man die natürliche DNA-Sequenz, die für die Aminosäuren +15 bis +243 des menschlichen Proapolipoproteins A-I codiert, verbindet stromabwärts von einem synthetischen DNA-Fragment der Sequenz (nur ein Strang dargestellt): 5' - ATGAGACATTTCTGGCAGCAGGACGAACCTCCACAATCTCCTTGGGATAGAGTTAAGGACTTG - 3' das für die Aminosäuren -6 bis +14 des menschlichen Proapolipoproteins A-I codiert und ein zusätzliches ATG-Codon zur Initiation der Translation und modifizierte Codons für die Reste der Aminosäuren -6, -1, +1, +3, +4, +5, +6, +7, +10, + 11 and +14 umfaßt.

2. Verfahren zur Herstellung eines replizierbareren Klonierungsvektors, dadurch gekennzeichnet, daß man die gemäß dem Verfahren nach Anspruch 1 hergestellte rekombinante DNA-Sequenz in einen geeigneten Vektor einfügt.

3. Verfahren zur Herstellung eines Expressionsvektors, dadurch gekennzeichnet, daß man auf operative Weise die gemäß dem Verfahren nach Anspruch 1 hergestellte rekombinante DNA-Sequenz mit einer regulatorischen DNA-Sequenz, die die Expression der rekombinanten DNA-Sequenz steuert, verbindet.

4. Verfahren nach Anspruch 3, dadurch gekennzeichnet, daß die regulatorische DNA-Sequenz den Bereich des Promotors P_{L} des Phagen lambda umfaßt.

5. Verfahren nach Anspruch 3, dadurch gekennzeichnet, daß die rekombinante DNA-Sequenz stromab - wärts mit der DNA-Sequenz der Beta-Galactosidase fusioniert ist und daß die regulatorische DNA-Sequenz den Bereich des Promotors lac von E. coli umfaßt.

6. Verfahren nach Anspruch 3, dadurch gekennzeichnet, daß die regulatorische DNA-Sequenz die Bereiche des Promotors und des Terminators der Transkription von ARG3 der Hefe umfaßt.

7. Verfahren nach Anspruch 3, dadurch gekennzeichnet, daß die rekombinante DNA-Sequenz ohne ATG - Codon stromabwärts von der DNA - Sequenz des Signalpeptids des OmpA - Proteins von E. coli fusioniert ist und daß die regulatorische DNA-Sequenz den Promotor Ipp, den Promotor-Operator lac und die Sequenz lac 1 des Repressors lac umfaßt.

8. Verfahren nach Anspruch 3, dadurch gekennzeichnet, daß die regulatorische DNA-Sequenz den Bereich des Promotors des Polyhedrin - Gens des Baculovirus umfaßt.

9. Verfahren zur Herstellung einer Zelle oder eines Mikroorganismus, dadurch gekennzeichnet, daß man eine Zelle oder einen Mikroorganismus mit einem Expressionsvektor transformiert, der nach einem Verfahren wie in einem der Ansprüche 3 bis 8 beschrieben hergestellt wurde.

10. Zellkultur oder Mikroorganismus, transformiert mit einem Expressionsvektor, der nach einem Verfahren wie in einem der Ansprüche 3 bis 8 beschrieben hergestellt wurde.

11. Verfahren zur Herstellung des menschlichen Proapolipoproteins A-I, dadurch gekennzeichnet, daß man unter geeigneten Kulturbedingungen eine transformierte Zelle oder Mikroorganismus gemaß Anspruch 10 kultiviert und, daß man das so hergestellte menschliche Proapolipoprotein A-I gewinnt.
